# EUROPEAN PATENT APPLICATION

(11) **EP 3 238 724 A2**
(43) Date of publication of application: **01.11.2017**
(21) Application number: 17173833.9
(22) Date of filing: 31.05.2013
(51) Int. Cl.: A61K 31/575, A61K 31/4745, A61K 31/565, A61P 25/28, A61P 5/46, A61K 31/22, A61K 31/7048, A61K 45/06, A61P 25/16, A61K 31/19, A61K 31/215

(54) **CHEMICAL SUPPRESSORS OF NEUROTOXICITY IN SYNUCLEINOPATHIC DISEASES**

(30) Priority: 01.06.2012 US 201261654284 P
(62) Divisional of application: 13797996.9
(71) Applicant: Oxalys Pharmaceuticals, Toronto, ON M1S 2L4 (CA)
(72) Inventor: SEPP, Katharine Julia, Toronto, Ontario M1S 2L4 (CA); SCHULTE, Joost, Toronto, Ontario M1S 2L4 (CA)
(74) Representative: Abel & Imray

(57) **Abstract**

Glycyrrhetinic acid analogs for use in treating neurodegenerative alpha-synuclein aggregation diseases and disorders such as Parkinson's disease.

## Description

### PRIORITY

This application claims priority to U.S. Provisional Application Ser. No. 61/654,284 filed June 1, 2012, herein incorporated in its entirety.

### FIELD OF THE INVENTION

This invention relates to the biological and medical fields. In some aspects, the invention relates to the field of neurodegenerative alpha-synuclein aggregation diseases and disorders, for example, the field of Parkinson's disease.

### BACKGROUND OF THE INVENTION

Parkinson's disease (PD) is a neurodegenerative disorder for which there are only symptomatic treatments. There are many causative factors that lead to PD, including inherited gene mutations, exposure to environmental toxins, and prior head trauma. A hallmark of PD is the accumulation of alpha-synuclein (aSYN) protein into Lewy bodies in neurons. Aggregation of aSYN leads to a number of neurodegenerative disorders, collectively termed 'synucleinopathies', which are thought to share pathological mechanisms. Excessive amounts of aSYN is thought to overwhelm systems for protein clearance, and thus interfere with normal cell functioning. A resultant cascade of neurotoxic effects occurs, which includes mitochondrial dysfunction and oxidative injury (Shults (2006) PNAS 103(6):1661-8). In PD, over time, there is a progressive loss of striatal dopamine, leading to motor features of the disease. While current treatments to replace dopamine are effective in treating motor control symptoms of the disease, they do not address the underlying pathological processes of the disease. Thus neuronal cell death occurs, most notably in the substantia nigra pars compacta. With further progression of the disease, neuronal loss becomes more widespread in the brain and leads to dementia. Thus there is an unmet need for treatments that can prevent neurotoxicity so that the underlying pathological effects of PD and related synucleinopathies can be slowed, halted, or reversed.

### SUMMARY OF THE INVENTION

Some aspects of this invention relate to compounds and compositions useful in the treatment of a synucleinopathy disease or disorder, for example, Parkinson's disease (PD). Some embodiments of this invention provide compounds and compositions that ameliorate a phenotype associated with synucleinopathy disorder, for example, increased aSYN protein aggregation, or pathologic changes in cell morphology. Some embodiments of this invention provide compounds and compositions that ameliorate a phenotype associated with synucleinopathy disorder without displaying significant cytotoxic or cytostatic characteristics, and without affecting tissue homeostasis or cell differentiation patterns. Some embodiments of this invention provide compounds and compositions for the treatment of a synucleinopathy disorder.

Some aspects of this invention relate to methods of treatment of a synucleinopathy disorder. For example, some embodiments provide a method for treating a synucleinopathy disease or disorder, comprising administering to a subject having or being at risk of having a synucleinopathy disease or disorder (e.g, suspected of having a synucleinopathy disease or disorder, or carrying a mutation of a gene implicated in a synucleinopathy disease or disorder, or having been exposed to toxins implicated in a synucleinopathy disease or disorder) an effective amount of carbenoxolone, or an analog, salt, or solvate thereof. In some embodiments, the synucleinopathy disease or disorder is Parkinson's disease (PD), Parkinson's disease with dementia (PDD), dementia with Lewy bodies (DLB), Lewy body variant of Alzheimer's disease, Alzheimer's disease (AD), Multiple System Atrophy (MSA), Down syndrome, Progressive Supranuclear palsy (PSP), Corticobasal degeneration (CBD), Shy-Drager syndrome, Striatonigral degeneration, Olivopontocerebellar atrophy, Pure autonomic failure, Prion disease, Neurodegeneration with brain iron accumulation type 1 (NBIA1), Frontotemporal dementia (FTD)/Pick's disease, Parkinsonism dementia complex /Amyotrophic lateral sclerosis (PDC/ALS) of Guam, amyotrophic lateral sclerosis (ALS), or traumatic brain injury. In some embodiments, the synucleinopathy disease or disorder is a mutation or copy number variation in the human *SNCA, LRRK2, PARK2, PARK7, PINK1, Parkin, DJ1, ATP13A2, PLA2G6, FBXO7, UCHL1, GIGYF2, HTRA2, EIF4G1, GBA, MAPT, BST1, GAK, APP, PS1, PS2, SOD1, P102L, 6-OPRI, E200K, PLA2G6, PANK2,* or *FTL* gene. In some embodiments, the synucleinopathy disease or disorder is an aneuploidy of human chromosome 21. In some embodiments, the subject expresses a mutant protein encoded by the *SNCA, LRRK2, PARK2, PARK7, PINK1, Parkin, DJ1, ATP13A2, PLA2G6, FBXO7, UCHL1, GIGYF2, HTRA2, EIF4G1, GBA, MAPT, BST1, GAK, APP, PS1, PS2, SOD1, P102L, 6-OPRI, E200K, PLA2G6, PANK2,* or *FTL* gene. In some embodiments, the subject has been exposed to neurotoxic compounds or elements including paraquat, rotenone, maneb, manganese, 1-methyl 4-phenyl 1,2,3,6-tetrahydropyridine (MPTP), reserpine, thorazine, toluene, n-hexane, carbon disulfide, carbon monoxide, mercury, cyanide, copper, lead, trichloroethylene, perchloroethylene, or 2,4-dichlorophenoxyacetic acid. In some embodiments, the synucleinopathy disease or disorder is PD. In some embodiments, the subject has been exposed to neurotoxic compounds.

In some embodiments, the subject has experienced head trauma. In some embodiments, the synucleinopathy disease or disorder is a brain degeneration syndrome that may be caused by a head trauma history. For example, the synucleinopathy disease or disorder may be chronic traumatic encephalopathy. In some embodiments, the subjects having a synucleinopathy disease or disorder are carriers of a SNCA Rep1 polymorphsim. This population is more susceptible to dementia associated with head trauma and are thus treated in some preferred embodiments of the present invention. In some embodiments, the subject is a human subject. In some embodiments, the carbenoxolone is administered orally. In some embodiments, the compound is administered at a dose of about 10 mg/day to about 10000 mg/day. In some embodiments, the compound is administered at a dose of about 1 mg/day to about 1000 mg/day. In some embodiments, the compound is administered at a dose of about 5mg/day to about 300 mg/day. In some embodiments, the method further comprises assessing the subject for symptoms of the synucleinopathy disease or disorder after administration of a compound and adjusting the dosage based on the assessment. In some embodiments, the subject exhibits a symptom associated with the synucleinopathy disease or disorder. In some embodiments, the method comprises maintaining or decreasing the dosage of the compound, if the subject exhibits a desired change in a symptom associated with the synucleinopathy disease or disorder. In some embodiments, the method comprises increasing the dosage, if the subject exhibits no desired change in a symptom associated with the synucleinopathy disease or disorder. In some embodiments, the subject does not exhibit a clinically manifest symptom of the synucleinopathy disease or disorder. In some embodiments, the clinically manifested symptom is an impairment in motor function, an impairment in cognitive function, an behavioral impairment, a functional impairment, or an impairment in Total Functional Capacity (TFC), either alone or in any combination thereof. In some embodiments, the subject exhibits an elevated glucocorticoid level. In some embodiments, the elevated glucocorticoid level is an elevated cortisol level. In some embodiments, the elevated cortisol level is a blood plasma level of more than 350nmol/L. In some embodiments, the elevated cortisol level is a blood plasma level of more than 700nmol/L. In some embodiments, the carbenoxolone, or analog, salt, or solvate thereof, is administered in an amount effective to reduce the elevated glucocorticoid level. In some embodiments, the carbenoxolone, or analog, salt, or solvate thereof, is administered in an amount effective to reduce the elevated glucocorticoid level to a level observed or expected in a healthy subject. In some embodiments, the carbenoxolone, or an analog, salt, or solvate thereof is administered to the subject based on the subject exhibiting an elevated glucocorticoid level. In some embodiments, the carbenoxolone, or an analog, salt, or solvate thereof is administered to the subject based on the subject exhibiting an elevated cortisol level.

In some embodiments, a method for treating a synucleinopathy disease or disorder is provided, comprising administering to a subject having or being at risk of having a synucleinopathy disease or disorder (e.g., a subject having or suspected of having a synucleinopathy disease or disorder, or carrying a mutation of a gene implicated in a synucleinopathy disease or disorder, or carrying a copy number variation of a gene implicated in a synucleinopathy disease or disorder, or having an aneuploidy of chromosome 21, or having exposure to neurotoxic chemicals, or having experienced head trauma) an effective amount of a compound chosen from the group of camptothecin, 10-hydroxycamptothecin, topotecan, irinotecan, 18β-glycyrrhetinic acid, glycyrrhetinic acid analog, carbenoxolone, etoposide, or an analog, salt, or solvate of any of these compounds, Topoisomerase I inhibitor, Topoisomerase II inhibitor, Topoisomerase III inhibitor, Topoisomerase IIIα inhibitor, or Topoisomerase IIIβ inhibitor, either alone, or in any combination.

In some embodiments, Cushing's Syndrome may be treated using the glycyrrhetinic acid and glycyrrhetinic acid analogs as provided herein. The chronic elevated levels of cortisol in a patient with Cushing's Syndrome may be lowered by the methods as described herein. In some embodiments, there is provided a method of treating chronic traumatic encephalopathy. Chronic traumatic encephalopathy is an example of a brain degeneration syndrome that may be caused by head trauma history that may be treated using the methods as described herein.

In some embodiments, the subject is a non-human mammal. In some embodiments, the subject is a human.

Some aspects of this invention provide methods for the use of the agents, compounds, molecules, and compositions in the preparation of a medicament, particularly a medicament for the treatment of synucleinopathy diseases, for example, PD, are also provided.

Additional aspects, embodiments, advantages, features, and uses of the invention will become apparent from the following detailed description of non-limiting embodiments of the invention when considered in conjunction with the accompanying drawings.

### BRIEF DESCRIPTION OF THE DRAWINGS

**Figures 1A** - **IE.** Analysis of compound treatment in neurons expressing human mutant alpha-synuclein (aSYN) in Drosophila and rats. Image features of primary neurons quantified with Cellomics ArrayScan software show improved neurite morphology profiles for Carbenoxolone (30 nM) and camptothecin-OH treated (30 nM) cultures, away from the DMSO-treated negative control values, and towards the morphology profiles of wild type (Elav wt) cultures.
   Figures 1A-1C. Primary neurons co-expressing human mutant aSYN A30P (red) and GFP (green) using the Elav transgene driver. Figure 1A is an image of DMSO vehicle-treated negative control cultures showing highly branched and short neurites, indicative of neurotoxicity. Figure 1 B is an image of Carbenoxolone-treated cultures (60 nM) showing longer neurites and reduced neuritic branching as compared to DMSO controls. Figure 1C is an image of Topotecan-treated cultures (60 nM) showing longer neurites and reduced neuritic branching as compared to DMSO controls.
   Figure ID is a chart wherein DMSO vehicle treated cultures show 15240±799.8 um(Mean ± SEM; n=18) total neurite length per well, whereas camptothecin-OH treated cultures show 20170 ± 1430um total neurite length per well (Mean ± SEM; n=4), with statistical significance P < 0.05 (asterisk).
   Figure IE is a chart wherein DMSO vehicle-treated cultures show 4.416 ± 0.2622 total branches per well (Mean ± SEM; n=16), whereas carbenoxolone-treated cultures show 3.198 ± 0.1825 total branches per well (Mean ± SEM; n=4), with statistical significance P < 0.05 (asterisk).
**Figure 2.** Liquid culture compound administration assay to aSYN expressing larvae. In liquid culture, wild type control (Elav wt), or aSYN expressing larvae are untreated (negative control, aSYN), or administered DMSO vehicle (negative control, aSYN DMSO), Camptothecin-OH (topoisomerase I inhibitor, aSYN CPT-OH), or Dexrazoxane (topoisomerase II alpha inhibitor, aSYN DEX) at a range of concentrations. Concentrations for 'hi': 15 uM; 'med': 0.15 uM, 'lo': 0.0015uM. After 4 days of treatment in liquid culture, the 'hi' dose of Camptothecin-OH yielded 100% survival (n=5), and the 'med' dose of Dexrazoxane yielded 75% survival (n=9), whereas untreated aSYN and DMSO treated larvae had 13% (n=69) and 14% (n=7) survival respectively.
**Figures 3A -3B.** Quantitative immunoblot analysis of aSYN expression in AAV-aSYN rat models of Parkinson's disease synucleinopathy. Rats received intra-nigral administration of AAV1/2A53T α-Synuclein to induce neuropathology and then three weeks later received continuous delivery (osmotic pump) of topotecan (TPT, 37.4 ug/h) or vehicle into their third ventricle for an additional 3 weeks. Mass-spec performed on cortex confirmed presence of TPT (n=3: 6809, 967, and 399 ng TPT/g tissue) and these samples were subjected to aSyn Quantitative Western Blotting.
   Figure 3A is an immunoblot image of the 'TPT hi': rat with highest levels of TPT in cortex (6809 ng TPT/g tissue). aSyn levels are normalized to β-actin. Vehicle control is on the left. Figure 3B is a chart comparing treatment to control. Compared to vehicle control, the three rats with an average of 2725 ng TPT/g tissue showed an approximate 2-fold reduction in aSYN as normalized to beta-Actin ('TPT' middle white bar), and the rat with the highest level of TPT in brain tissue (6809 ng TPT/g tissue) showed an approximate 5-fold reduction in aSYN as normalized to beta-Actin.
**Figure 4.** Exemplary structures of compounds that suppress or are expected to suppress neurotoxicity in PD mouse models.

### DETAILED DESCRIPTION OF CERTAIN EMBODIMENTS

Synucleinopathies are a class of neurologic disorders, characterized by an abnormal accumulation of alpha-synuclein (aSYN) protein into toxic oligomers and aggregates. The aggregates are downstream products of earlier pathological events, such as aberrant gene function, exposure to chemical toxins, and head trauma. Excessive aSYN oligomers are thought to overwhelm normal mechanisms for protein clearance, they impair mitochondrial function, and disrupt cellular architecture, leading to cell injury and death in vulnerable neuronal and glial populations. Exemplary synucleinopathy diseases and disorders are listed in Table 1 below. For references, see Galvin JE, Lee VMY, Trojanowski JQ (2001) Synucleinopathies: clinical and pathological implications. Arch Neurol 58:186-190; Schulte C, Gasser T (2011) Genetic basis of Parkinson's disease: inheritance, penetrance, and expression. Application Clin Genet 4:67-80; Campbell TN, Choy FYM (2012) Gaucher disease and the synucleinopathies: refining the relationship. Orphanet Journal of Rare Diseases 7:12; Ma Q-L, Chan P, Yoshii M, Ueda K (2003) α-Synuclein aggregation and neurodegenerative diseases. J Alzheimer's Dis 5:139-148; Liu Y, Yang H (2005) Environmental toxins and α-synuclein in Parkinson's disease. Mol Neurol 31:273-282; Goldman SM, Kamel F, Ross GW, Jewell SA, Bhudhikanok GS, Umbach D, Marras C, Hauser RA, Jankovic J, Factor SA, Bressman S, Lyons KE, Meng C, Korell M, Roucoux DF, Hoppin JA, Sandler DP, Langston JW, Tanner CM (2012) Head injury, α-synuclein Rep1, and Parkinson's disease. Ann Neurol 7:40-48.

**TABLE 1.Exemplary synucleinopathies, causative factors, and aSYN pathology.**

| Disease | Disease subclass | Causative factors | Location of aSYN pathology |
|---|---|---|---|
| Parkinson's disease (PD), Parkinson's disease with dementia (PDD) | Familial | Mutations: SNCA, LRRK2, PARK2, PARK7, PINK1, Parkin, DJ1, ATP13A2, PLA2G6, FBXO7, UCHL1, GIGYF2, HTRA2, EIF4G1, GBA, MAPT, BST1, GAK, HLA | Widespread aggregates in neurons and astrocytes |
| | Sporadic | Toxins: paraquat, rotenone, maneb, manganese, MPTP, reserpine, thorazine, toluene, n-hexane, carbon disulfide, carbon monoxide, mercury, cyanide, copper, lead, tricholorethylene, | |
| | | perchloroethylene,2,4-dichlorophenoxyacetic acid. Head trauma. | |
| Alzheimer disease (AD) | Familial | Mutations: APP, PS1, PS2 | Non-amyloid beta component of plaques |
| | Sporadic | | Non-amyloid beta component of plaques |
| | Lewy body variant | | Lewy bodies |
| Dementia with Lewy bodies (DLB) | Pure Lewy body dementia | Mutations: SNCA, LRRK2, GBA. | Widespread aggregates in neurons and astrocytes |
| Multiple system atrophy (MSA) | Parkinsonia n subtype (MSA-P) | Unknown | Glial, neuronal cytoplasmic inclusions |
| | Cerebellar dysfunction subtype (MSA-C) | Unknown | Glial, neuronal cytoplasmic inclusions |
| Down syndrome with AD | | Trisomy 21 | Lewy bodies in amygdala |
| Progressive Supranuclear palsy (PSP) | | Unknown | Neurofibrillary tangles, glial inclusions |
| Corticobasal degeneration (CBD) | | Unknown | Neurofibrillary tangles, glial inclusions |
| Pure autonomic failure | | Unknown | Parasympathetic neuronal inclusions |
| Prion disease | Sporadic | Unknown | Misfolded isoforms in Prion protein plaques |
| | Iatrogenic | Growth hormone administration, cadaver sourced medical implants | |
| | Inherited | Mutations (e.g. P102L, 6-OPRI, E200K) | |
| | New variant Creutzfeldt-Jakob | Consumption of infected meat products | |
| Neurodegeneration with brain iron accumulation, type 1 (NBIA1) | | Mutations: PLA2G6, PANK2, FTL | Spheroid axonal swellings, neurites |
| Frontotemporal dementia (FTD)/Pick's disease | | Unknown | Frontal cerebral cortex, nucleus basalis of Meynert, Pick bodies |
| Parkinsonism dementia complex /Amyotrophic lateral sclerosis | | Environmental influences | Aggregates in amygdala |
| (PDC/ALS) of Guam | | | |
| Amyotrophic lateral sclerosis (ALS) | | | Spinal cord, neuronal cytoplasm |
| Traumatic brain injury | Acute | SNCA Rep1 promoter variation, injury | Axonal swellings |

Since aSYN aggregation is a common fundamental pathological aspect of neurodegeneration, it is important to develop therapeutic interventions to clear aggregated aSYN and prevent further accumulation. Here, we identify therapeutic compounds, which may effectively clear aSYN from neural cells. We also identify therapeutic compounds, which may provide significant neuroprotection to prevent aSYN accumulation in stressed neurons. These compounds will be validated preclinically in mouse PD models for their capacity for neuroprotection and aSYN clearance.

### Methods and compositions for treating a synucleinopathy disease or disorder

Some aspects of the invention relate to compounds and compositions for the treatment of synucleinopathy diseases or disorders, for example, the diseases and disorders described in Table 1. In some embodiments, a compound is provided that is expected to modulate a phenotype observed in a synucleinopathy-associated disease in a desirable way. For example, in some embodiments, a compound or composition is provided that ameliorates aggregation of aSYN. In some embodiments, the compound does not have significant cytotoxic side effects on the target cells. In some embodiments, the compound does have tolerable cytotoxic side effects on the target cells. In some embodiments, a compound or composition is provided that ameliorates a morphological change observed in cells expressing aSYN. In some embodiments, a compound or composition is provided that protects cells from genetic mutations that cause aSYN aggregation. In some embodiments, a compound or composition protects cells from chemical toxins that cause synucleinopathies. In some embodiments, a compound or composition protects cells from pathological processes that follow as a result of head trauma.

Not wishing to be bound by theory, we hypothesize that topoisomerase inhibitors are able to ameliorate cellular phenotypes typical for synucleinopathy diseases or disorders, for example, Parkinson's disease. In particular, the camptothecin class of topoisomerase inhibitors are able to ameliorate cellular phenotypes typical for synucleinopathy disease, for example, Parkinson's disease, while not exhibiting significant cytotoxicity in the target cells.

### Camptothecin and analogs

Camptothecin is a cytotoxic quinoline alkaloid which inhibits the DNA enzyme topoisomerase I (also known as topo I, topoisomerase 1, topo 1, top 1, or top I). Because camptothecin can induce adverse side reaction in some subjects and at some dosages, various camptothecin derivatives have been developed. Camptothecins are in clinical use for the treatment of cancer. Currently, two camptothecins, topotecan and irinotecan, are FDA approved and are used in the clinic for cancer treatment.

Some aspects of this invention provide a method for treating a synucleinopathy disease or disorder, comprising administering to a subject having or suspected of having a synucleinopathy disorder or disease an effective amount of a compound provided herein. In some embodiments, the compound being administered is camptothecin or a camptothecin derivative.

In some embodiments, the camptothecin or camptothecin derivative is a compound described by Formula 1: 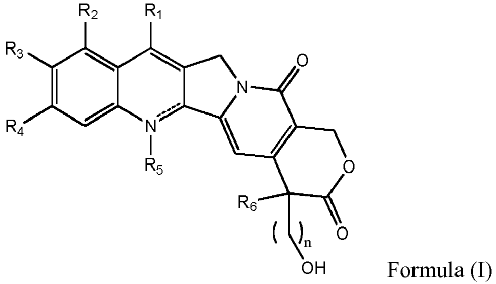 wherein
R₁ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{A}; =O; -C(=O)R_{A}; -CO₂R_{A}; -CN; -SCN; -SR_{A}; -SOR_{A}; -SO₂R_{A}; -NO₂; -N(R_{A})₂;-NHC(O)R_{A}; or -C(R_{A})₃; wherein each occurrence of R_{A} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
R₂ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{B}; =O; -C(=O)R_{B}; -CO₂R_{B}; -CN; -SCN; -SR_{B}; -SORB; -SO₂R_{B}; -NO₂; -N(R_{B})₂;-NHC(O)R_{B}; or -C(R_{B})₃; wherein each occurrence of R_{B} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
R₃ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{C}; =O; -C(=O)R_{C}; -CO₂R_{C}; -CN; -SCN; -SR_{C}; -SOR_{C}; -SO₂R_{C}; -NO₂; -N(R_{C})₂;-NHC(O)R_{C}; or -C(R_{C})₃; wherein each occurrence of R_{C} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
R₄ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{D}; =O; -C(=O)R_{D}; -CO₂R_{D}; -CN; -SCN; -SR_{D}; -SOR_{D}; -SO₂R_{D}; -NO₂; -N(R_{D})₂;-NHC(O)R_{D}; or -C(R_{D})₃; wherein each occurrence of R_{D} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
or wherein R₂ and R₃ together or R₃ and R₄ together are -X-(C(R_{E})₂)ₘ-X-, wherein each occurrence of X is independently O, N, or S; each occurrence of R_{E} is independently hydrogen, a protecting group, alkyl, alkoxy, aryloxy, alkylthio, arylthio, amino, alkylamino, dialkylamino, heteroaryloxy, or heteroarylthio; and m is 1, 2, 3, 4, or 5;
R₅ is hydrogen; cyclic or acyclic, substituted or unsubstituted, branched or unbranched aliphatic; cyclic or acyclic, substituted or unsubstituted, branched or unbranched heteroaliphatic; -C(=O)R_{F}; -CO₂R_{F}; or -C(RF)₃; wherein each occurrence of R_{F} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
R₆ is a substituted or unsubstituted, branched or unbranched aliphatic; or cyclic or acyclic, substituted or unsubstituted, branched or unbranched heteroaliphatic;
n is 0, 1, 2, 3, or 4; or
a salt, or solvate thereof.

In some embodiments, R₃ and R₄ together are -O-(CH₂)ₘ-O-. In some embodiments, R6 is methyl, ethyl, or propyl. In some embodiments R6 is ethyl. In some embodiments, the compound of Formula (I) has the structure of Formula (V):

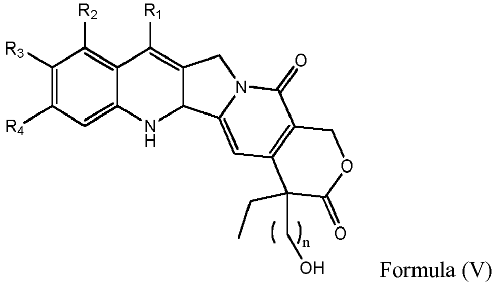

In some embodiments, the camptothecin administered to a subject having or suspected of having a synucleinopathy disorder or disease is substituted at position 7, 9, 10 and/or 11 (C atom having a covalent bond to R1, R2, R3, and R4, respectively). For example, in some embodiments, the camptothecin is 10-Hydroxycamptothecin. In some embodiments, the camptothecin comprises an enlarged lactone ring, for example, a lactone ring that is enlarged by one methylene unit (e.g., homocamptothecin).

In some embodiments, the camptothecin comprises an electron-withdrawing group, for example, an amino, nitro, bromo or chloro group, at position 9 and/or 10 and/or a hydroxyl group at position 10 and/or 11. In some embodiments, the camptothecin is a hexacyclic camptothecin analog, comprising, for example, a methylenedioxy or ethylenedioxy group connected between position 10 and 11 to form a 5 or 6 membered ring. In some embodiments, the camptothecin is Lurtotecan, a 10, 11-ethylenedioxy camptothecin analogue with a 4-methylpiperazino-methylene at position 7.

Some exemplary camptothecin derivatives that are useful according to some embodiments of the invention are given in Table 2 below.

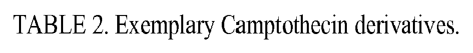

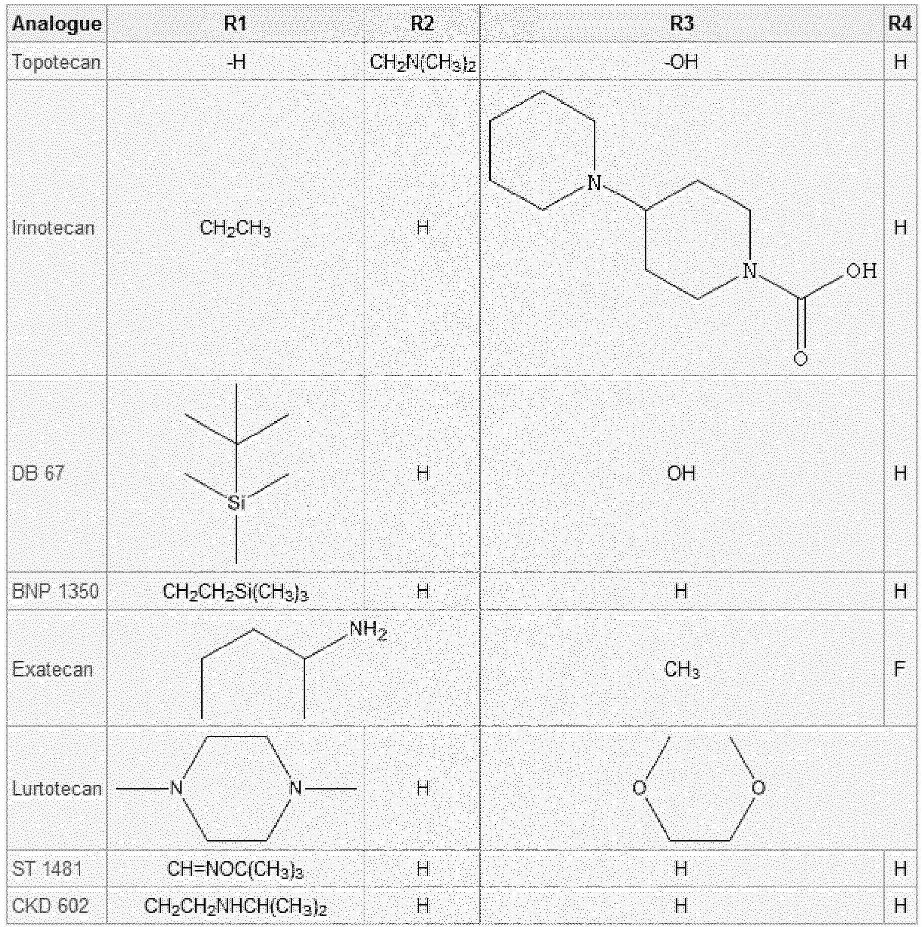

Some aspects of this invention provide a method for treating a synucleinopathy disease or disorder, comprising administering to a subject having or suspected of having a synucleinopathy disorder or disease a compound provided herein. In some embodiments, the method comprises administering a compound provided in Table 3. In some embodiments, the compound is chosen from the group of camptothecin, 10-hydroxycamptothecin, topotecan, irinotecan, 18β-glycyrrhetinic acid, glycyrrhetinic acid analog, carbenoxolone, etoposide, or a pharmaceutically acceptable analog, salt, or solvate of any of these compounds.

### Glycyrrhetinic acid Analogs

Glycyrrhetinic acid and glycyrrhetinic analogs are described by the structure of Formula (III) 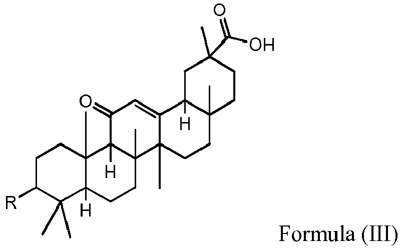 wherein R is OH, -OR_{A}; -CO₂R_{A}; -SO₂R_{A}; wherein each occurrence of R_{A} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio. Salts and solvates of glycyrrhetinic acid and glycyrrhetinic analogs are also contemplated. In some embodiments, R is OH, OCO-CH₃, - OCO-(CH₂)₂-COOH, or a salt or solvate thereof.

The glycyrrhetinic acid analogs as described by the structure as Formula (III) are also described herein as carbenoxolone analogs.

Useful carbenoxolone analogs and derivatives will be apparent to those of skill in the art. Such useful analogs and derivatives include, but are not limited to, BX24, oleanoic acid sodium hydrogen succinate (OSS), and cicloxolone. Useful carbenoxolone analogs further include, but are not limited to deuterated carbenoxolone analogs, in which one or more H atoms of the carbenoxolone molecule, or of a carbenoxolone analog molecule, is substituted with a deuterium atom.

Carbenoxolone is a glycyrrhetinic acid derivative that is also known as (3β)-3-[(3-carboxy-propanoyl)oxy]-11-oxoolean-12-en-30-oic acid; as (3β,20β)-3-(3-carboxy-1-oxopropoxy)-11-oxoolean-12-en-29-oic acid; as (2S,4aS,6aS,6bR, 8aR, 10S, 12aS, 12bR, 14bR) -10-(3-carboxy-propanoyloxy)-2,4a,6a,6b,9,9,12a-heptamethyl-13-oxo-1,2,3,4,4a,5,6,6a,6b,7,8,8a,9,10,11, 12, 12a, 12b, 13, 14b- icosahydropicene-2-carboxylic acid; as butanedioic acid, mono[(3beta)-30-hydroxy-11,30-dioxoolean-12-en-3-yl] ester; as glycerrhetinic acid hydrogen succinate; as glycyrrhetic acid hydrogen succinate; as enoxolone succinate; and as CBX.

Salts of carbenoxolone, or of its analogs or derivatives, that are useful according to some aspects of this invention are well known to those of skill in the art and include, but are not limited to sodium and disodium salts (e.g., carbenoxolone sodium and carbenoxolone disodium salts). Other salts that are useful according to some aspects of the invention include pharmaceutically acceptable salts (e.g., pharmaceutically acceptable carbenoxolone salts).

The structure of carbenoxolone is well known to those of skill in the art and an exemplary representation of the structure of carbenoxolone is provided in Formula (IV) :

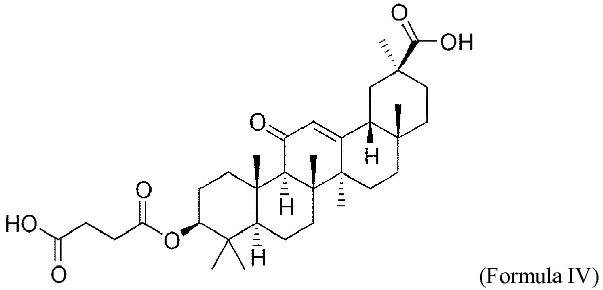

Carbenoxolone is in clinical use, for example, for the treatment of oesophagal ulceration, inflammation, and for the treatment of oral and perioral lesions. Not wishing to be bound by theory, it is hypothesized that carbenoxolone is particularly useful for treating a synucleinopathy disease or disorder.

In some embodiments, a method for treating a synucleinopathy disease or disorder is provided, comprising administering to a subject having or suspected of having a synucleinopathy disorder or disease carbenoxolone, or a carbenoxolone analog or derivative, or a pharmaceutically acceptable salt of carbenoxolone or a carbenoxolone analog or derivative. In some embodiments, the method includes administering to a subject having or suspected of having Parkinson's disease an amount of carbenoxolone, or of a carbenoxolone analog or derivative, that is sufficient, either alone or in combination with additional administered amounts, to achieve a reduction in the aggregation of aSYN protein, a reduction in the number or size of inclusion bodies, a normalization of brain tissue homeostasis (e.g. improved survival of neuronal cells and/or reduction in reactive astrocytes), an improvement in cognitive and motor function, and/or a slowing or reversal of a personality change commonly associated with PD.

In some embodiments, a method for treating a synucleinopathy disease or disorder is provided, comprising administering to a subject having or suspected of having a synucleinopathy disorder or disease, for example, PD, carbenoxolone, or a carbenoxolone analog or derivative, or a pharmaceutically acceptable salt of carbenoxolone or a carbenoxolone analog or derivative, via an enteral administration route. For example, in some embodiments, carbenoxolone, an analog or derivative, or salt thereof, is administered orally to the subject.

Formulations of carbenoxolone, or a carbenoxolone analog or derivative, for oral administration are well known to those of skill in the art and include, but are not limited to those formulations of carbenoxolone in the drugs used under the trade names BIOGASTRONE^{™}; BIOPLEX^{™}; BIORAL^{™}; CARBOSAN^{™}; DUOGASTRONE^{™}; GASTRAUSIL^{™}; HERPESAN^{™}; NEOGEL^{™}; ROWADERMAT^{™}; SANODIN^{™}; ULCUS-TABLINEN^{™}, and PYROGASTRONE^{™}. Additional suitable formulations of carbenoxolone or a carbenoxolone analog or derivative, for oral administration to a subject having or suspected of having a synucleinopathy disorder or disease will be apparent to those of skill in the art and include, but are not limited to formulation in capsules, tablets, lozenges, suspensions, syrups, elixirs, and emulsions.

### Dosing

In some embodiments, a method for treating a synucleinopathy disease or disorder is provided, comprising administering to a subject having or suspected of having a synucleinopathy disorder or disease, for example, PD, a compound described herein, for example, camptothecin, 10-hydroxycamptothecin, topotecan, irinotecan, 18β-glycyrrhetinic acid, glycyrrhetinic acid analog, carbenoxolone, etoposide, or a pharmaceutically acceptable analog, salt, or solvate of any of these compounds at a dosage that is sufficient to achieve a desirable clinical result in the subject, but is non-toxic to the subject. In some embodiments, the compound, analog, salt, or solvate is administered to a subject having or suspected of having a synucleinopathy disease at a dose in the range of 0.1 mg to 10,000 mg per day. In some embodiments, the compound, analog, salt, or solvate is administered to a subject having or suspected of having a synucleinopathy disease at a dose of more than 10,000 mg per day.

For example, in some embodiments, a compound described herein, for example, camptothecin, 10-hydroxycamptothecin, topotecan, irinotecan, 18β-glycyrrhetinic acid, glycyrrhetinic acid analog, carbenoxolone, etoposide, or a pharmaceutically acceptable analog, salt, or solvate of any of these compounds is administered to a subject having or suspected of having a synucleinopathy disease at a dose of about 10 mg/day, about 20 mg/day, about 30 mg/day, about 40 mg/day, about 50 mg/day, about 60 mg/day, about 70 mg/day, about 80 mg/day, about 90 mg/day, about 100 mg/day, about 150 mg/day, about 200 mg/day, about 250 mg/day, about 300 mg/day, about 350 mg/day, about 400 mg/day, about 450 mg/day, about 500 mg/day, about 550 mg/day, about 600 mg/day, about 650 mg/day, about 700 mg/day, about 750 mg/day, about 800 mg/day, about 850 mg/day, about 900 mg/day, about 950 mg/day, about 1000 mg/day, about 1050 mg/day, about 1100 mg/day, about 1150 mg/day, about 1200 mg/day, about 1250 mg/day, about 1300 mg/day, about 1350 mg/day, about 1400 mg/day, about 1450 mg/day, about 1500 mg/day, about 1550 mg/day, about 1600 mg/day, about 1650 mg/day, about 1700 mg/day, about 1750 mg/day, about 1800 mg/day, about 1850 mg/day, about 1900 mg/day, about 1950 mg/day, or about 2000 mg/day.

In some embodiments, a compound described herein, for example, camptothecin, 10-hydroxycamptothecin, topotecan, irinotecan, 18β-glycyrrhetinic acid, glycyrrhetinic acid analog, carbenoxolone, etoposide, or a pharmaceutically acceptable analog, salt, or solvate of any of these compounds is administered to a subject having or suspected of having a synucleinopathy disease at a dose that is determined based on the body weight of the subject (e.g., mg of compound (e.g., carbenoxolone) per kg of body weight of the subject), for example, at a dose of about 0.01 mg/kg, about 0.02 mg/kg, about 0.025 mg/kg, about 0.03 mg/kg, about 0.04 mg/kg, about 0.05 mg/kg, about 0.06 mg/kg, about 0.07 mg/kg, about 0.08 mg/kg, about 0.09 mg/kg, about 0.1mg/kg, about 0.2 mg/kg, about 0.25mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, or about 1 mg/kg. In some embodiments, the dosage is at more than 1mg/kg. In some embodiments, for example, in some embodiments, in which carbenoxolone, or an analog, salt, or solvate thereof, is administered to a subject, the amounts in mg/kg provided herein are given as a daily dose, e.g., as 0.01 mg/kg/day, 0.02 mg/kg/day, *etc.*

In some embodiments, a compound described herein is administered to a subject having or suspected of having a synucleinopathy disease orally, for example, via a pill or tablet.

In some embodiments, oral administration is performed once, twice, or three times daily.

In some embodiments, a method for treating a synucleinopathy disease or disorder is provided, comprising administering to a subject having or suspected of having a synucleinopathy disorder or disease, for example, PD, carbenoxolone, or a carbenoxolone analog or derivative, or a pharmaceutically acceptable salt of carbenoxolone or a carbenoxolone analog or derivative, via a parenteral administration route, for example, via subcutaneous, intramuscular, intraperitoneal, or intravenous injection. Some methods and formulation for parenteral administration of carbenoxolone or carbenoxolone analogs and derivatives are provided herein and additional methods and formulations are well known to those of skill in the art.

In some embodiments, a method for treating a synucleinopathy disease or disorder is provided, comprising administering to a subject having or suspected of having a synucleinopathy disorder or disease, for example, PD, carbenoxolone, or a carbenoxolone analog or derivative, or a pharmaceutically acceptable salt of carbenoxolone or of a carbenoxolone analog or derivative, at a dosage that is sufficient to achieve a desirable clinical result in the subject, but is non-toxic to the subject. In some embodiments, carbenoxolone or a carbenoxolone analog or derivative, or a salt thereof, is administered to a subject having or suspected of having a synucleinopathy disease at a dose in the range of 0.1 mg to 10,000 mg per day. In some embodiments, carbenoxolone or a carbenoxolone analog or derivative, or a salt thereof, is administered to a subject having or suspected of having a synucleinopathy disease at a dose of more than 10,000 mg per day.

In some embodiments, a compound described herein (e.g., a compound of Formula I, II, or III) is administered to a subject carrying a mutation associated with a synucleinopathy disease (e.g., a pathologic mutation or copy number change of a gene product described in Table 1), before a clinical symptom of the synucleinopathy disease manifests. For example, some embodiments provide methods of administering an 18β-glycyrrhetinic acid analog, for example, carbenoxolone, to a subject expressing a familial *SNCA* A53T mutation, before the patient manifests a clinical symptom of PD. In some embodiments, the compound is administered based on the subject carrying the synucleinopathy-associated gene mutation. In some embodiments, the compound is administered based on prior exposure to synucleinopathy-linked chemicals. In some embodiments, the compound is administered based on prior head trauma. In some embodiments, the compound is administered based on prior head trauma and genetic mutation in the *SNCA* Rep1 promoter region. In some embodiments, the compound is administered to prevent or delay the onset of, or mitigate the severity of a clinical symptom of the synucleinopathy disease.

In some such pre-symptomatic treatment embodiments, the administration of the compound, for example, of an 18β-glycyrrhetinic acid analog (e.g., carbenoxolone), prevents the onset of clinical symptoms of the disease (e.g., PD) in the subject, while in other embodiments, the onset of a clinical manifest symptom of the disease is merely delayed as compared to an untreated subject. In some pre-symptomatic treatment embodiments, the administration of the compound, for example, of the 18β-glycyrrhetinic acid analog (e.g., carbenoxolone), mitigates the severity of a symptom of the disease, for example, the severity of a motor, behavioral, or cognitive impairment associated with the synucleinopathy disease. In some embodiments, the administration of the compound prior to clinical symptom manifestation delays the progression of the synucleinopathy disease once symptoms develop.

In some embodiments, a compound described herein is chronically administered to a subject carrying a pathologic synucleinopathy-associated mutation, or expressing an abnormal copy number of a synucleinopathy-associated gene, for example, for at least 1 month, at least 2 months, at least 3 months, at least 4 months, at least 5 months, at least 6 months, at least 12 months, at least 18 months, at least 2 years, at least 3 years, at least 4 years, at least 5 years, at least 6 years, at least 7 years, at least 8 years, at least 9 years, at least 10 years, at least 15 years, at least 20 years, at least 25 years, at least 30 years, at least 35 years, at least 40 years, or at least 50 years. In some such embodiments, the compound is administered at a dose that is non-toxic in long-term administration. In some such embodiments, the compound is administered at the highest, non-toxic dose. In some embodiments, the compound is administered at the lowest dose effective to prevent, delay, or mitigate the severity of a clinically manifest symptom of the disease. In some embodiments, the compound, for example, carbenoxolone, is administered at a dose recommended by the manufacturer, or approved or accepted by those of skill in the art to be safe for long-term administration.

In some embodiments, the pre-symptomatic treatment methods provided herein further comprise monitoring the subject for a clinical manifestation of a symptom associated with the synucleinopathy disorder. Clinical symptoms of synucleinopathy diseases and methods for their assessment in subjects having or suspected to have such a disease are well known to those of skill in the art. For example, clinical symptoms of PD and methods for their diagnosis and quantification have been published in the Unified Parkinson's Disease Rating Scale (UPDRS Development Committee, Fahn S, primary author). The Unified Parkinson's Disease Rating Scale (UPDRS): status and recommendations. Mov Disord 2003;18:738-50; the entire contents of which are incorporated herein by reference).

Some of the compounds disclosed herein, for example, some 18β-glycyrrhetinic acid analogs (e.g., carbenoxolone), can decrease the level of a glucocorticoid, for example, a cortisol level, when administered to a subject, for example, to a subject exhibiting an elevated glucocorticoid level. Neuroendocrine studies have shown that there is defective control of the hypothalamus-pituitary-adrenal gland (HPA) axis in PD (Rabey et al., 1990; Volpi et al., 1994; Volpi et al., 1997) and elevated cortisol levels have been detected in patients (Hartmann et al., 1997; Skogar et al., 2011)(Charlett et al., 1998). Stress research has shown that chronic cortisol exposure is neurotoxic and causes brain shrinkage particularly in the hippocampus (Lupien et al., 1998). The toxic effects of chronic cortisol exposure are not fully understood but they may be related to altered glucocorticoid receptor signaling. Chronic cortisol exposure is known to suppress neuronal brain derived neurotrophic factor (BDNF) production, exacerbate neuronal excitotoxicity, and to oppose the action of insulin (McEwen, 2010). While the underlying disease mechanisms of synucleinopathies are not understood, it is thought that decreased BDNF levels, excitotoxicity, and insulin resistance are significant exacerbating features of PD pathology (Howells et al., 2000; Dong et al., 2009)(Aviles-Olmos et al., 2012). Together, these results suggest that restoring cortisol balance in PD patients has the potential for both immediate and long-term benefits. 11-beta-hydroxysteroid dehydrogenase 1 (HSD1) is a brain enzyme that regulates the production of cortisol in the brain. HSD1 is expressed widely in the forebrain (including the basal ganglia), hippocampus and cerebellum by both neurons and glia. Although the majority of cortisol produced in the body is generated by the adrenal glands, after activation of the HPA-axis, cortisol is unstable and is rapidly catabolized into the inactive analogue cortisone soon after it is released into the blood. The action of brain HSD1 then locally converts inactive cortisone to cortisol to sustain the effects of the active molecule for extended periods of time. One example of a compound disclosed herein that can decrease elevated corticosteroid levels is carbenoxolone.

Carbenoxolone is a steroid-like molecule with lipophilic properties that reduces levels of active cortisol in the brain through inhibition of HSD1. Beneficial effects of HSD1 inhibition are seen from mouse HSD1 knockouts which show enhanced cognition in aged animals (Yau et al., 2007). Similarly, effects have been observed in rodent and human studies after pharmacological inhibition of the enzyme. For references, see Skogar O, Fall PA, Hallgren G, Lokk J, Bringer B, Carlsson M, Lennartsson U, Sandbjork H, Tornhage CJ (2011) Diurnal salivary cortisol concentrations in Parkinson's disease: increased total secretion and morning cortisol concentrations. Int J Gen Med 4:5651-569; Charlett A, Dobbs RJ, Purkiss AG, Wright DJ, Peterson DW, Weller C, Dobbs SM (1998) Cortisol is higher in parkinsonism and associated with gait deficit. Acta Neurol Scand 92:77-85; Djamshidian A, O'Sullivan SS, Papadopoulos A, Bassett P, Shaw K, Averbeck BB, Lees A (2011) Salivary cortisol levels in Parkinson's disease and its correlation to risk behavior. J Neurol Neurosurg Psychiatry 82:1107-1111; Lupien SJ, de Leon M, de Santi S, Convit A, Tarshish C, Nair NP, Thakur M, McEwen BS, Hauger RL, Meaney MJ (1998) Cortisol levels during human aging predict hippocampal atrophy and memory deficits. Nat Neurosci 1:69-73; Dong XX, Wang Y, Qin ZH (2009) Molecular mechanisms of excitotoxicity and their relevance to pathogenesis of neurodegenerative disease. Acta pharmacol Sinica 30:379-387; Bosco D, Plastino M, Cristiano D, Colica C, Ermio C, De Bartolo M, Mungari P, Fonte G, Consoli D, Consoli A, Fava A (2012) Dementia is associated with insulin resistance in patients with Parkinson's disease. J Neurol Sci 315:39-43; Morris JK, Bomhoff GL, Gorres BK, Davis VA, Kim J, Lee PP, Brooks WM, Gerhardt GA, Geiger PC, Stanford JA (2011) Insulin resistance impairs nigrostriatal dopamine function. Exp Neurol 231:171-180; Howells DW, Pirritt MJ, Wong JY, Batchelor PE, Kalnins R, Hughes AJ, Donnan GA (2000) Reduced BDNF mRNA expression in the Parkinson's disease substantia nigra. Exp Neurol 166:127-135; Wang M (2011) Inhibitors of 11beta-hydroxysteroid dehydrogenase type 1 in antidiabetic therapy. Handb Exp Pharmacol 203:127-146; McEwen BS (2010) Stress, sex, and neural adaptation to a changing environment: mechanisms of neuronal remodeling. Ann NY Acad Sci Sep;1204 Suppl:E38-59; Schulte J, Sepp KJ, Wu C, Hong P, Littleton JT (2011) High-content chemical and RNAi screens for suppressors of neurotoxicity in a Huntington's disease model. PLoS One 6(8):e23841. Epub 2011 Aug 31; and Yau JL, McNair KM, Noble J, Brownstein D, Hibbernd C, Morton N, Mullins JJ, Morris RG, Cobb S, Seckl JR (2007) Enhanced hippocampal long-term potentiation and spatial learning in aged 11beta-hydroxysteroid dehydrogenase type 1 knock-out mice. J Neurosci 27:10487-10496; the entire contents of each of which are incorporated herein by reference.

It is important to note that previous results demonstrating *in vivo* rescue of toxicity from the aggregation-prone polyglutamine-expanded Huntingtin protein by carbenoxolone in Drosophila (Schulte et al., 2011) is surprising in this regard, because there is no ortholog of the 11beta-HSD1 and also no ortholog of a glucocorticoid receptor in Drosophila. Further, administering a different 11beta-HSD1 inhibitor to the Drosophila model failed to produce the same result as carbenoxolone. Accordingly, carbenoxolone likely effects suppression of the toxicity of human mutant Huntingtin protein in the Drosophila model by an unknown, 11beta-HSD1-independent pathway.

Given that carbenoxolone and its 18β-glycyrrhetinic acid analog were both shown to protect against polyglutamine protein aggregation in a Huntington's disease (HD) model (Schulte et al., 2011), it cannot be assumed that carbenoxolone analogs are widely applicable to all neurodegenerative diseases. In contrast to the toxic nuclear Huntingtin protein aggregates observed in Huntington's disease, the accumulations of aSYN in synucleinopathies are cytoplasmic and localize largely to neuritic processes. Nuclear proteasomal degradation of proteins is different from that of the cytoplasm, since nuclear proteolysis assists in DNA repair and nuclear-specific proteasomal subunits are used (von Mikecz, 2006). Furthermore, polyglutamine aggregates and aSYN aggregates sequester different protein types. Sequestration of different proteins would interfere with different cellular pathways, leading to different cellular pathologies. This is likely a reason why different neuronal subpopulations are affected in different neurodegerative diseases.

It is also found that the majority of compounds identified so far in drug discovery screening are not broadly applicable to a variety of neurodegenerative disease classes, with the exception of compounds that target the heat shock response (Westerheide and Morimoto, 2005). The 18β-glycyrrhetinic acid and its analogs are not known to target the heat shock response, and both compounds showed significantly higher capacity to suppress polyglutamine toxicity in an HD model as compared to known heat shock-targeted drugs radicicol and geldanamycin (Schulte et al., 2011).

Both carbenoxolone and 18β-glycyrrhetinic acid also have gap-junction blocking activity (Juszczak and Swiergiel, 2009)(Takeuchi et al., 2011), which would be therapeutically beneficial for a broad spectrum of neurodegenerative diseases. However, these compounds do not cross the blood-brain-barrier at high enough concentrations to achieve this effect *in vivo* (Takeuchi et al., 2011). Therefore, based on current data on the 18β-glycyrrhetinic acid activities, the neuroprotective mechanisms responsible for in vivo suppression of polyglutamine toxicity in a previous humanized Drosophila Huntington's disease model (Schulte et al., 2011) are not understood. The activity of 18β-glycyrrhetinic acid and its analogs may suppress toxicity of aSYN in synucleinopathy diseases and disorders, thus the present invention provides novel disease-modifying therapeutics.

If the neuroprotective mechanism of carbenoxolone as described herein is more broadly applicable to protein aggregation neurodegenerative disorders, carbenoxolone may be useful as a prevention in pre-symptomatic individuals carrying mutations linked to synucleinopathy diseases since early dosing has been shown to extend Drosophila HD model lifespan and improves motor control (Schulte et al., 2011). For the same reason, carbenoxolone may also be useful as a prevention in pre-symptomatic individuals who have been exposed to chemical toxins associated with synucleinopathy diseases, as well as pre-symptomatic individuals who have a history of head trauma.

Some aspects of this invention are based on the recognition that, controlling cortisol levels has clear benefits to pre-symptomatic as well as symptomatic subjects having a synucleinopathy disease, or carrying a mutation associated with a synucleinopathy disease, or carrying a copy number variation of a gene associated with a synucleinopathy disease, or having a history of synucleinopathy-associated chemical toxin exposure, or having a history of head trauma. In some embodiments, a compound described herein, for example, carbenoxolone or an analog thereof, is administered to a subject carrying a mutation in a gene that is associated with a synucleinopathy disease or disorder (see, e.g., Table 1 for exemplary genes), or carrying a copy number variation of a gene associated with a synucleinopathy disease, or an aneuploidy of chromosome 21, or having a history of synucleinopathy-associated chemical toxin exposure (see, e.g., Table 1 for exemplary toxins), or having a history of head trauma and exhibiting an elevated level of a glucocorticoid, for example, of cortisol. For example, in some embodiments, a compound described herein, for example, carbenoxolone or an analog thereof, is administered to a subject carrying a mutation in a gene that is associated with PD, or carrying a copy number variation of a gene associated with HD, or having a history of PD-associated chemical toxin exposure, or having a history of head trauma, and exhibiting an elevated level of a glucocorticoid, for example, of cortisol. In some embodiments, the compound is administered to treat a symptom of a synucleinopathy disease, e.g., PD, for example, an impairment in motor or cognitive function (e.g., tremor or memory loss). Some aspects of this invention are based on the recognition that the psychiatric and cognitive disturbances of synucleinopathy diseases may be largely due to chronic high cortisol in patients, and that at least part of the beneficial effect of some of the compounds described herein, e.g., carbenoxolone, is due to the reduction of cortisol levels effected by those compounds. This is consistent with the data from Drosophila (Schulte et al., 2011), which shows that carbenoxolone improves motor coordination, which is unexpected given known cortisol effects on psychiatric and cognitive health. Chronically elevated cortisol is linked to hippocampal-dependent memory deficits and a decline in hippocampal volume. This is observed in hypercortisolemic (chronic high cortisol) but otherwise normal human, hypercortisolemia rodent models, and PD patients (see, e.g., Lupien SJ, de Leon M, de Santi S, Convit A, Tarshish C, Nair NP, Thakur M, McEwen BS, Hauger RL, Meaney MJ (1998) Cortisol levels during human aging predict hippocampal atrophy and memory deficits. Nat Neurosci 1:69-73; Skogar O, Fall PA, Hallgren G, Lokk J, Bringer B, Carlsson M, Lennartsson U, Sandbjork H, Tornhage CJ (2011) Diurnal salivary cortisol concentrations in Parkinson's disease: increased total secretion and morning cortisol concentrations. Int J Gen Med 4:5651-569; Djamshidian A, O'Sullivan SS, Papadopoulos A, Bassett P, Shaw K, Averbeck BB, Lees A (2011) Salivary cortisol levels in Parkinson's disease and its correlation to risk behavior. J Neurol Neurosurg Psychiatry 82:1107-1111; Rabey JM, Scharf M, Oberman Z, Zohar M, Graff E (1990) Cortisol, ACTH, and beta-endorphin after dexamethasone administration in Parkinson's dementia Biol Psychiatry 15:581-591; the entire contents of each of which are incorporated herein by reference). Psychiatric effects that are linked to chronic elevated cortisol are: depression, anxiety, insomnia, compulsive behavior, and mood swings, and these changes are observed in both PD patients and hypercortisolemic but otherwise normal humans (see, e.g., Dubrovsky B (1993) effects of adrenal cortex hormones on limbic structures: some experimental and clinical correlations related to depression. J Psychiatry Neurosci 18:4-16; Bruin VM, Bittencourt LR (2012) Sleep-wake disturbances in Parkinson's disease: current evidence regarding diagnostic and therapeutic decisions. Eur Neurol 67:257-267; Aarsland D, Pahlhagen S, Ballard CG, Ehrt U, Svenningsson P (2011) Depression in Parkinson disease - epidemiology, mechanisms and management. Nat Rev Neurol 8:35-47; Leroi I, Andrews M, McDonald K, Harbishettar V, Elliott R, Byrne EJ, Burns A (2011) Apathy and impulse control disorders in Parkinson's disease: a direct comparison. Parkinsonism Relat Disord 18;198-203; Prediger RD, Matheus FC, Schwarzbold ML, Lima MM, Vital MA (2012) Anxiety in Parkinson's disease: a critical review of experimental and clinical studies. Neuropharmacology 62:115-124; Nissenbaum H, Quinn NP, Brown RG, Toone B, Gotham AM, Marsden CD (1987) Mood swings associated with the 'on-off phenomenon in Parkinson's disease. Psychol Med 17:899-904; the entire contents of each of which are incorporated herein by reference).

Administration of a compound described herein to a subject exposed to a chemical toxin known to cause Parkinson's disease symptoms e.g., administration of 1-methyl-4-phenyl-1,2,3,6-tetrahydropyridine (MPTP), before the synucleinopathy disease symptoms appears, can prevent or delayed onset of such symptoms in vivo. Accordingly, the compounds described herein are useful for the treatment of symptomatic subjects, but also for administration to pre-symptomatic subjects. In some embodiments, a compound described herein, for example, carbenoxolone or an analog thereof, is administered to a subject carrying a mutation in a gene that is associated with a synucleinopathy disease or disorder, or carrying a copy number variation of a gene that is associated with a synucleinopathy disease or disorder (see, e.g., Table 1 for exemplary genes), or exposed to a chemical toxin linked to a synucleinopathy disease or disorder, or with a history of head trauma, before a clinical symptom of the disease or disorder, for example, a motor impairment, cognitive impairment, behavioral impairment, restriction of independence, functional impairment, or an impairment in Total Functional Capacity (TFC) is clinically manifest. For example, in some embodiments, a compound described herein, for example, carbenoxolone or an analog or salt thereof, is administered to a subject carrying a mutation in the *SNCA* gene that is associated with PD, or a copy number variation of the *SNCA* gene implicated in PD, or having been exposed to the chemical toxin rotenone, or having experienced a head trauma before a clinical symptom of PD, for example, a motor impairment, cognitive impairment, behavioral impairment, restriction of independence, functional impairment, or and impairment in Total Functional Capacity (TFC) is clinically manifest. Accordingly, some embodiments provide methods of treating a synucleinopathy disease or disorder in pre-symptomatic subjects. (e.g., subjects that do not show outward signs of tremor, psychiatric disturbances or cognitive decline), in order to prevent or delay the onset of a symptom of the disease or disorder.

In some embodiments, a compound described herein, for example, carbenoxolone or an analog thereof, is administered to a subject carrying a mutation in a gene that is associated with a synucleinopathy disease or disorder, or carrying a copy number variation of a gene that is associated with a synucleinopathy disease or disorder (see, e.g., Table 1 for exemplary genes), or exposed to a chemical toxin linked to a synucleinopathy disease or disorder, or with a history of head trauma, that exhibits an elevated glucocorticoid level, for example, an elevated cortisol level, before a clinical symptom of the disease or disorder, for example, a motor impairment, cognitive impairment, behavioral impairment, restriction of independence, functional impairment, or and impairment in Total Functional Capacity (TFC) is clinically manifest. For example, in some embodiments, a compound described herein, for example, carbenoxolone or an analog or salt thereof, is administered to a subject carrying a mutation in the *SNCA* gene that is associated with PD, or a copy number variation of the *SNCA* gene implicated in PD, or having been exposed to the chemical toxin rotenone, or having experienced a head trauma before a clinical symptom of PD, and exhibiting an elevated level of a glucocorticoid, for example, of cortisol, before a clinical symptom of PD, for example, a motor impairment, cognitive impairment, behavioral impairment, restriction of independence, functional impairment, or an impairment in Total Functional Capacity (TFC) is clinically manifest. Such treatment of pre-symptomatic subjects with elevated glucocorticoid levels allows for the prevention or the delay of the onset of symptoms associated with the disease or disorder.

The structure of numerous glucocorticoids, for example, of cortisol, as well as methods of measuring the level of glucocorticoids in a subject, and normal and elevated glucocorticoid levels, e.g., levels of cortisol present or expected to be present in a healthy subject or above the range deemed normal for a healthy subject, respectively, are well known to those of skill in the art. Elevated cortisol levels according to some aspects of this invention can be measured in body fluids including, but not limited to, urine, saliva, blood, blood plasma, and cerebrospinal fluid. Methods for such measurements are well known to those of skill in the art, and the invention is not limited in this regard.

For example, in some embodiments, normal blood plasma cortisol levels are between 70nmol/l - 700nmol/l (between 2.5 ug/dL - 25 µg/dL), or between 70nmol/l - 350nmol/l (between 2.5 µg/dL - 12.5 µg/dL), depending on the parameters of the assay. Methods for measuring cortisol levels, e.g., in the blood or urine of a subject, and normal ranges in addition to the ranges provided herein, are known to those of skill in the art and the invention is not limited in this respect. In some embodiments, a level above the normal range of cortisol levels, for example, a blood plasma cortisol level of more than 350nmol/l, 400nmol/l, 500nmol/l, 600nmol/l, 700nmol/l (e.g., a level of more than 750nmol/l, more than 800nmol/l, more than 900nmol/l, more than 1µmol/l, more than 2µmol/l, more than 2.5µmol/l, more than 5µmol/l, more than 10µmol/l, more than 20µmol/l, more than 25µmol/l, more than 50µmol/l, more than 100µmol/l, or more than 500µmol/l) is an elevated cortisol level. In some embodiments, a compound described herein, for example, carbenoxolone or an analog or salt thereof, is administered to the subject in an amount effective to reduce an elevated glucocorticoid level, for example, an elevated cortisol level, in the subject. In some embodiments, the 18β-glycyrrhetinic acid or an analog thereof, for example, carbenoxolone, is administered to the subject in an amount effective to reduce an elevated glucocorticoid level, for example, an elevated cortisol level, in the subject to a level that is less than 90%, less than 80%, less than 75%, less than 70%, less than 60%, less than 50%, less than 40%, less than 30%, less than 25%, less than 20%, less than 10%, less than 5%, less than 2.5%, less than 1%, or less than 0.1% of the level exhibited by the subject prior to administration of the 18β-glycyrrhetinic acid or an analog thereof. In some embodiments, the 18β-glycyrrhetinic acid or an analog thereof, for example, carbenoxolone, is administered to the subject in an amount effective to reduce an elevated glucocorticoid level, for example, an elevated cortisol level, in the subject to a non-pathogenic level, or a level not deemed to be elevated, or a level expected to be present in a healthy subject. For example, in some embodiments, carbenoxolone is administered to a subject carrying a mutation of the *SNCA* gene, and exhibiting an elevated cortisol level (e.g., a blood plasma level of more than 350nmol/l or more than 700nmol/l, but not exhibiting a clinical symptom of HD, in an amount effective to reduce the cortisol level to a normal level (e.g., to a blood plasma level within the range of 70-350nnmol/l or 70-700nmol/l). In some embodiments, the cortisol level is monitored in the subject after administration of carbenoxolone, and the dosage is adjusted, e.g., increased if the cortisol level is determined to still be elevated, or decreased if the cortisol level is lower than desired (e.g., lower than 70nmol/l). In some embodiments, the lowest dose of carbenoxolone required to maintain a normal cortisol level (e.g., a blood plasma cortisol level of 70nmol/l - 700nmol/l) is determined by repeated administration of carbenoxolone to the subject and monitoring of the cortisol level. In some embodiments, the lowest dose required to maintain a normal cortisol level is used for long-term administration in the subject.

In some embodiments, carbenoxolone or a carbenoxolone analog or derivative is administered to a subject having or suspected of having a synucleinopathy disease or disorder, or carrying a synucleinopathy-associated gene mutation, or carrying a copy number variation of a gene that is associated with a synucleinopathy disease or disorder (see, e.g., Table 1 for exemplary genes), or having or suspected of having been exposed to a chemical toxin linked to a synucleinopathy disease or disorder, or with a history of head trauma, in combination with one or more additional drug. In some embodiments, the one or more additional drug is a compound described herein, for example, camptothecin, 10-hydroxycamptothecin, topotecan, irinotecan, 18β-glycyrrhetinic acid, and/or etoposide, or a pharmaceutically acceptable analog, salt, or solvate of any of these compounds. In some embodiments, the one or more additional drug is a compound identified in Formula 1. In some embodiments, the one or more additional drug is a drug that ameliorates an undesired side-effect of carbenoxolone or the analog, salt, or solvate thereof that is administered. For example, in some embodiments, the one or more additional drug is a drug that ameliorates hypertension, hypoalkaemia, and/or electrolyte retention (e.g., sodium retention). Non-limiting examples of such drugs are antihypertensive drugs, potassium supplements, and diuretics. Antihypertensive drugs, potassium supplements, and diuretics as well as effective amounts and suitable administration routes of such drugs are well known to those of skill in the art and the invention is not limited in this respect. For example, in some embodiments, a combination of carbenoxolone and an antihypertensive drug are administered to a subject having or suspected of having a synucleinopathy disease. In some embodiments, a combination of carbenoxolone and a potassium salt are administered to a subject having or suspected of having a synucleinopathy disease. In some embodiments, a combination of carbenoxolone and a diuretic drug are administered to a subject having or suspected of having a synucleinopathy disease.

Some aspects of this invention provide a method for treating a synucleinopathy disease or disorder, comprising administering to a subject having or suspected of having a synucleinopathy disorder or disease a Topoisomerase 1 inhibitor, Topoisomerase 2 inhibitor, Topoisomerase 3 inhibitor, or Topoisomerase 3α inhibitor. Exemplary inhibitors of these types are provided herein and additional inhibitors are well known to those of skill in the art and include, for example, RNAi agents (e.g. siRNA, shRNA, antisense RNA), small molecule compounds, antibodies, or antigen-binding fragments thereof, aptamers, and adnectins.

In some embodiments, the method comprises administering a single compound provided herein, for example, a single compound provided in Table 3. In some embodiments, the method comprises administering a combination of compounds as provided herein, or a combination of one or more compounds as provided herein with a compound known in the art to be useful in the treatment of a synucleinopathy disease or disorder.

In some embodiments, the synucleinopathy disease or disorder is Parkinson's disease (PD), Parkinson's disease with dementia (PDD), Alzheimer's disease (AD), dementia with Lewy bodies (DLB), multiple system atrophy (MSA), Down syndrome with AD, progressive supranuclear palsy (PSP), cortocobasal degeneration (CBD), pure autonomic failure, prion disease, neurodegeneration with brain iron accumulation type 1 (NBIA1), frontotemporal dementia (FTD), Parkinsonism dementia complex/amyotrophic lateral sclerosis of Guam (PDC/ALS), or amyotrophic lateral sclerosis (ALS). In some embodiments, the subject has experienced head trauma and/or has been exposed to chemical toxins associated with synucleinopathy diseases or disorders, such as paraquat, rotenone, maneb, manganese, MPTP, reserpine, thorazine, toluene, n-hexane, carbon disulfide, carbon monoxide, mercury, cyanide, copper, lead, tricholorethylene, perchloroethylene, 2,4-dichlorophenoxyacetic acid. In some embodiments, the method of treating comprises administering a compound as provided herein to a subject diagnosed with any of the aforementioned diseases. In some embodiments, the method of treating comprises administering a compound as described herein to a subject based on the subject being diagnosed with the disease or based on the subject being suspected to have the disease.

In some embodiments, the synucleinopathy disease or disorder is causally related to a mutation or copy number variation in the *SNCA, LRRK2, PARK2, PARK7, PINK1, Parkin, DJ1, ATP13A2, PLA2G6, FBXO7, UCHL1, GIGYF2, HTRA2, EIF4G1, GBA, MAPT, BST1, GAK, APP, PS1, PS2, SOD1, P102L, 6-OPRI, E200K, PLA2G6, PANK2,* or *FTL* gene. In some embodiments, the synucleinopathy disease or disorder is causally related to a third human chromosome 21. In some embodiments, the method comprises administering a compound provided herein to a subject based on the subject being diagnosed with having a synucleinopathy genetic defect, for example, a mutation, chromosomal aneuploidy, or gene copy number variation in any of the aforementioned genes or chromosome.

Some aspects of this invention provide methods for treating a subject. In some embodiments, the subject is human. In some embodiments, the subject is a non-human mammal, for example, a non-human primate, a mouse, a rat, a pig, a dog, or a cat. In some embodiments, the subject is a non-mammal, for example, an insect, or a fish, an amphibian, or a reptile.

Some aspects of this invention also provide methods for preparing a medicament or a formulation for the treatment of a synucleinopathy disorder. In some embodiments, a compound or composition described herein is formulated for administration to a subject in need of such treatment.

Some aspects of the invention relate to methods of treating synucleinopathy diseases or disorders, or treating a subject carrying a synucleinopathy-associated genetic lesion, or treating a subject with previous history of exposure to chemical toxins that cause synucleinopathy, or treating a subject with previous history of head trauma prior to the manifestation of clinical symptoms of a synucleinopathy disease or disorder associated with the mutation or polypeptide. In some embodiments, a compound or composition as provided herein is administered to a subject having or suspected of having a synucleinopathy disease or disorder. In some embodiments, the compounds or compositions as provided herein are administered in an "effective amount". An "effective amount" in the context of treatment of a synucleinopathy disease or disorder is an amount of a compound or composition as described herein that alone, or together with further doses, produces a desired response, e.g. modulation of aSYN aggregation, decrease in aSYN expression, cell morphology, and/or amelioration of any functional symptoms associated with the synucleinopathy disease or disorder. For example, desired responses to treatment in the context of Parkinson's disease include, but are not limited to, a reduction in the aggregation of aSYN protein, reduction in cellular amounts of aSYN protein, reduction in the number or size of Lewy bodies, a normalization of brain tissue homeostasis (e.g. improved survival of neuronal cells and/or reduction in reactive astrocytes), an improvement in cognitive and motor function, and/or is slowing or reversal of the personality change commonly associated with PD.

In some embodiments, in the case of treating a particular disease or condition described herein the desired response is inhibiting the progression of the disease or condition. In some embodiments, this involves only slowing the progression of the disease temporarily, although more preferably, it involves halting the progression of the disease permanently. In some embodiments, the response of the subject to the administration of a compound provided herein is monitored by routine diagnostic methods known to one of ordinary skill in the art for the particular disease. In some embodiments, the desired response to treatment of the disease or condition is delaying the onset or even preventing the onset of the disease or condition, or reversing the physiological effects of the disease.

The effective amount will depend on the particular condition being treated, the severity of the condition, the individual patient parameters including age, physical condition, size and weight, the duration of the treatment, the nature of concurrent therapy (if any), the formulation of the compound, the specific route of administration and like factors within the knowledge and expertise of the health practitioner. These factors are well known to those of ordinary skill in the art and can be addressed with no more than routine experimentation. It is generally preferred that a maximum dose of the agent that modulates a synucleinopathy disease or disorder (alone or in combination with other therapeutic agents) be used, that is, the highest safe dose according to sound medical judgment. It will be understood by those of ordinary skill in the art, however, that a patient may insist upon a lower dose or tolerable dose for medical reasons, psychological reasons or for virtually any other reasons.

The pharmaceutical compositions used in the foregoing methods preferably are sterile and contain an effective amount of one or more compounds or compositions as described herein for producing the desired response in a unit of weight or volume suitable for administration to a patient.

The doses of compounds or compositions administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

Various modes of administration will be known to one of ordinary skill in the art which effectively deliver the compounds or compositions to a desired tissue, cell or bodily fluid. Administration includes: topical, intravenous, oral, intracavity, intrathecal, intrasynovial, buccal, sublingual, intranasal, transdermal, intravitreal, subcutaneous, intramuscular and intradermal administration. The invention is not limited by the particular modes of administration disclosed herein. Standard references in the art (e.g., *Remington's Pharmaceutical Sciences,* 18th edition, 1990) provide modes of administration and formulations for delivery of various pharmaceutical preparations and formulations in pharmaceutical carriers. Other protocols which are useful for the administration of compounds or compositions will be known to one of ordinary skill in the art, in which the dose amount, schedule of administration, sites of administration, mode of administration (e.g., intra-organ) and the like vary from those presented herein.

Administration to mammals other than humans of compounds or compositions, e.g. for testing purposes or veterinary therapeutic purposes, is carried out under substantially the same conditions as described above. It will be understood by one of ordinary skill in the art that this invention is applicable to both human and animal diseases that can be treated by the compounds or compositions as described herein. Thus this invention is intended to be used in husbandry and veterinary medicine as well as in human therapeutics.

In general, a therapeutically effective amount of a compound or composition provided herein typically varies from about 0.01 ng/kg to about 1000 µg/kg, preferably from about 0.1 ng/kg to about 200 µg/kg and most preferably from about 0.2 ng/kg to about 20 µg/kg, in one or more dose administrations daily, for one or more days. Lesser or greater amounts may be found to be therapeutically effective and thus also are useful in accordance with the invention.

The pharmaceutical preparations of the invention may be administered alone or in conjunction with standard treatment(s) of the disorders described herein, e.g., synucleinopathy diseases or disorders such as Parkinson's disease.

Pharmaceutical preparations of the invention are administered in effective amounts and in pharmaceutically-acceptable compositions. The term "pharmaceutically acceptable" means a non-toxic material that does not interfere with the effectiveness of the biological activity of the active ingredients. Such preparations may routinely contain salts, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts. Suitable pharmaceutically acceptable salts of compounds include acid addition salts which may, for example, be formed by mixing a solution of the compound with a solution of a pharmaceutically acceptable acid, such as hydrochloric acid, hydrobromic acid, sulfuric acid, fumaric acid, maleic acid, succinic acid, benzoic acid, acetic acid, citric acid, tartaric acid, phosphoric acid, carbonic acid, or the like. Where the compounds carry one or more acidic moieties, pharmaceutically acceptable salts may be formed by treatment of a solution of the compound with a solution of a pharmaceutically acceptable base, such as lithium hydroxide, sodium hydroxide, potassium hydroxide, tetraalkylammonium hydroxide, lithium carbonate, sodium carbonate, potassium carbonate, ammonia, alkylamines, or the like.

The compounds or compositions described herein may be combined, if desired, with a pharmaceutically-acceptable carrier. The term "pharmaceutically-acceptable carrier" as used herein means one or more compatible solid or liquid fillers, diluents or encapsulating substances which are suitable for administration into a human. The term "carrier" denotes an organic or inorganic ingredient, natural or synthetic, with which the active ingredient is combined to facilitate the application.

Pharmaceutically acceptable carriers include pharmaceutically acceptable salts, where the term "pharmaceutically acceptable salts" includes salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge et al., "Pharmaceutical Salts", Journal of Pharmaceutical Science, 1977, 66, 1-19). Certain specific compounds of the present invention contain both basic and acidic functionalities that allow the compounds to be converted into either base or acid addition salts. The components of the pharmaceutical compositions also are capable of being co-mingled with the compounds or compositions, and with each other, in a manner such that there is no interaction which would substantially impair the desired pharmaceutical efficacy.

The pharmaceutical compositions may contain suitable buffering agents, as described above, including: acetate, phosphate, citrate, glycine, borate, carbonate, bicarbonate, hydroxide (and other bases) and pharmaceutically acceptable salts of the foregoing compounds.

The pharmaceutical compositions also may contain, optionally, suitable preservatives, such as: benzalkonium chloride; chlorobutanol; parabens; and thimerosal.

The pharmaceutical compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active compound. Other compositions include suspensions in aqueous liquids or non-aqueous liquids such as a syrup, elixir or an emulsion.

Compositions suitable for parenteral administration may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or di-glycerides. In addition, fatty acids such as oleic acid may be used in the preparation of injectables. Carrier formulation suitable for oral, subcutaneous, intravenous, intramuscular, etc. administrations can be found in Remington's Pharmaceutical Sciences, Mack Publishing Co., Easton, PA.

A long-term sustained release implant also may be used for administration of the pharmaceutical agent composition. "Long-term" release, as used herein, means that the implant is constructed and arranged to deliver therapeutic levels of the active ingredient for at least 30 days, and preferably 60 days. Long-term sustained release implants are well known to those of ordinary skill in the art and include some of the release systems described above. Such implants can be particularly useful in treating conditions by placing the implant near portions of a subject affected by such activity, thereby effecting localized, high doses of the compounds of the invention.

As used herein, an "aliphatic" group is a straight or branched chain, or cyclic hydrocarbon radical, or combination thereof, which may be fully saturated, mono- or polyunsaturated and can include di- and multivalent radicals, having the number of carbon atoms designated (i.e. C₁-C₆ means one to six carbons). Examples of saturated hydrocarbon radicals include, but are not limited to, alkyl groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. In some embodiments, the aliphatic groups are C₁ - C₆ aliphatic groups. Examples of aliphatic groups include the short chain aliphatics such as methyl, ethyl, propyl, isopropyl, butyl, and pentyl and these aliphatics substituted with halogen, amino, and hydroxy groups. In some embodiments, the aliphatic group is a C₁ - C₆ alkyl group.

As used herein a "heteroaliphatic" group is, unless otherwise stated, a stable straight or branched chain, or cyclic hydrocarbon radical, or combinations thereof, consisting of the stated number of carbon atoms and at least one heteroatom selected from the group consisting of O, N, Si and S, and wherein the nitrogen, carbon and sulfur atoms may optionally be oxidized and the nitrogen heteroatom may optionally be quaternized. The heteroatom(s) O, N and S and Si may be placed at any interior position of the heteroalkyl group or at the position at which the alkyl group is attached to the remainder of the molecule. Examples include, but are not limited to, -CH₂-CH₂-O-CH₃, -CH₂-CH₂-NH-CH₃, -CH₂-CH₂-N(CH₃)-CH₃, -CH₂-S-CH₂-CH₃, -CH₂-CH₂, -S(O)-CH₃, -CH₂-CH₂-S(O)₂-CH₃, -CH=CH-O-CH₃, -Si(CH₃)₃, -CH₂-CH=N-OCH₃, and -CH=CH-N(CH₃)-CH₃. Up to two heteroatoms may be consecutive, such as, for example, -CH₂ -NH-OCH₃ and -CH₂-O-Si(CH₃)₃. Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini (e.g., alkyleneoxy, alkylenedioxy, alkyleneamino, alkylenediamino, and the like). The terms "heteroaliphatic" as well as "heteroalkyl" and "heteroalkylene" encompass poly(ethylene glycol) and its derivatives (see, for example, Shearwater Polymers Catalog, 2001). Still further, for alkylene and heteroalkylene linking groups, no orientation of the linking group is implied by the direction in which the formula of the linking group is written. For example, the formula -C(O)₂ R'- represents both -C(O)₂ R'- and -R'C(O)₂-. Examples of heteroaliphatic groups include methylpiperazino-methylene, trimethylsilyl-dimethyl methylene, and trimethylsilyl-ethylene. In some embodiments, the heteraliphatic group is a C₁ - C₆ alkyl group having at least one heteroatom intercalated into the alkyl chain or substituted onto the alkyl chain.

As used herein an "aryl" group is, unless otherwise stated, a substituted or unsubstituted polyunsaturated, aromatic, hydrocarbon substituent which can be a single ring or multiple rings (preferably from 1 to 3 rings) which are fused together or linked covalently.

The term "heteroaryl" refers to aryl groups (or rings) that contain from one to four heteroatoms selected from N, O, and S, wherein the nitrogen, carbon and sulfur atoms are optionally oxidized, and the nitrogen atom(s) are optionally quaternized. A heteroaryl group can be attached to the remainder of the molecule through a heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems are selected from the group of acceptable substituents described below. "Aryl" and "heteroaryl" also encompass ring systems in which one or more non-aromatic ring systems are fused, or otherwise bound, to an aryl or heteroaryl system. Examples of aryl and heteroaryl groups include benzene, naphthalene, indene, pyridine, pyrrole, benzofuran, and oxazine.

Substituents for the alkyl, and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) are generally referred to as "alkyl substituents" and "heteroalkyl substituents," respectively, and they can be one or more of a variety of groups selected from, but not limited to: -OR', =O, NR', =N-OR', -NR'R", -SR', -halogen, -SiR'R"R"', -OC(O)R', -C(O)R', -CO₂ R',-CONR'R", -OC(O)NR'R", -NR"C(O)R', -NR'-C(O)NR"R''', NR"C(O)₂ R', NR-C(NR'R"R''')=NR'''', -NR-C(NR'R")=NR''', -S(O)R', -S(O)₂ R', -S(O)₂NR'R", NRSO₂R',-CN and -NO₂ in a number ranging from zero to (2m'+1), where m' is the total number of carbon atoms in such radical. R', R", R''' and R'''' each preferably independently refer to hydrogen, substituted or unsubstituted heteroalkyl, substituted or unsubstituted aryl, e.g., aryl substituted with 1-3 halogens, substituted or unsubstituted alkyl, alkoxy or thioalkoxy groups, or arylalkyl groups. When a compound of the invention includes more than one R group, for example, each of the R groups is independently selected as are each R', R", R''' and R'''' groups when more than one of these groups is present. When R' and R" are attached to the same nitrogen atom, they can be combined with the nitrogen atom to form a 5-, 6-, or 7-membered ring. For example, -NR'R" is meant to include, but not be limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl (e.g., -CF₃ and -CH₂ CF₃) and acyl (e.g., -C(O)CH₃, -C(O)CF₃, -C(O)CH₂ OCH ₃, and the like).

Similar to the substituents described for the alkyl radical, the aryl substituents and heteroaryl substituents are generally referred to as "aryl substituents" and "heteroaryl substituents," respectively and are varied and selected from, for example: halogen, -OR', =O, =NR', =N-OR',-NR'R", -SR', -halogen, -SiR'R"R''', -OC(O)R', -C(O)R', -CO₂ R', -CONRR", -O-OC(O)NR'R", -NR"C(O)R', -NR'-C(O)NR"R''', NR"C(O)₂ R', -NR-C(NR'R")=NR''',-S(O)R', -S(O)₂ R', -S(O)₂NR'R", -NRSO₂ R', -CN and NO₂, -R', N₃, -CH(Ph)₂, fluoro(C ₁-C₄)alkoxy, and fluoro(C₁-C₄)alkyl, in a number ranging from zero to the total number of open valences on the aromatic ring system; and where R', R", R''' and R" " are preferably independently selected from hydrogen, (C₁-C₈)alkyl and heteroalkyl, unsubstituted aryl and heteroaryl, (unsubstituted aryl)-(C₁-C₄ )alkyl, and (unsubstituted aryl)oxy-(C₁-C₄)alkyl. When a compound of the invention includes more than one R group, for example, each of the R groups is independently selected as are each R', R", R''' and R'''' groups when more than one of these groups is present.

The term "solvate" refers to a crystal form where a stoichiometric or non-stoichiometric amount of solvent, or mixture of solvents, is incorporated into the crystal structure. Examples of solvents are water, acetone, ethanol, methanol, propanol, dichloromethane, etc.

As used herein, the term "subject" is a human or other animal, having or suspected of having a synucleinopathy disease or disorder. Thus, in some embodiments the subject will be in need of the therapeutic treatment as provided herein. Preferred patients are mammals. Examples of patients include but are not limited to, humans, horses, monkeys, dogs, cats, mice, rates, cows, pigs, goats and sheep.

As used herein, the terms "administering" or "administration" are intended to encompass all means for directly and indirectly delivering a compound to its intended site of action.

The term "therapeutically effective amount" refers to an amount effective, at dosages and for periods of time necessary, to achieve the desired therapeutic result. A therapeutically effective amount of the composition may vary according to factors such as the disease state, age, sex, and weight of the individual, and the ability of the pharmacological agent to elicit a desired response in the individual. A therapeutically effective amount is also one in which any toxic or detrimental effects of the pharmacological agent are outweighed by the therapeutically beneficial effects. For compounds described herein, the therapeutically effective amount can be initially determined from cell culture assays. Target plasma concentrations will be those concentrations of active compound(s) that are capable of inhibition cell growth or division. In preferred embodiments, the cellular activity is at least 25% inhibited. Target plasma concentrations of active compound(s) that are capable of inducing at least about 50%, 75%, or even 90% or higher inhibition of cellular activity are presently preferred. The percentage of inhibition of cellular activity in the patient can be monitored to assess the appropriateness of the plasma drug concentration achieved, and the dosage can be adjusted upwards or downwards to achieve the desired percentage of inhibition.

As is well known in the art, therapeutically effective amounts for use in humans can also be determined from animal models. For example, a dose for humans can be formulated to achieve a circulating concentration that has been found to be effective in animals. The dosage in humans can be adjusted by monitoring cellular inhibition and adjusting the dosage upwards or downwards, as described above.

A therapeutically effective dose can also be determined from human data for compounds which are known to exhibit similar pharmacological activities. The applied dose can be adjusted based on the relative bioavailability and potency of the administered compound as compared with the known compound.

The term "about" or "approximately" means within an acceptable error range for the particular value as determined by one of ordinary skill in the art, which will depend in part on how the value is measured or determined-e.g., the limitations of the measurement system, or the degree of precision required for a particular purpose. For example, "about" can mean within 1 or more than 1 standard deviations, as per the practice in the art. Alternatively, "about" can mean a range of up to 20%, preferably up to 10%, more preferably up to 5%, and more preferably still up to 1% of a given value. Where particular values are described in the application and claims, unless otherwise stated, the term "about" meaning within an acceptable error range for the particular value should be assumed.

As used herein and in the appended claims, the singular forms "a," "an," and "the," include plural referents unless the context clearly indicates otherwise. Thus, for example, reference to "a molecule" includes one or more of such molecules, "a resin" includes one or more of such different resins and reference to "the method" includes reference to equivalent steps and methods known to those of ordinary skill in the art that could be modified or substituted for the methods described herein.

While the above description provides examples and specific details of various embodiments, it will be appreciated that some features and/or functions of the described embodiments admit to modification without departing from the scope of the described embodiments. The above description is intended to be illustrative of the invention, the scope of which is limited only by the language of the claims appended hereto.

The function and advantage of these and other embodiments of the present invention will be more fully understood from the examples below. The following examples are intended to illustrate the benefits of the present invention, but do not exemplify the full scope of the invention.

### EXAMPLES

### EXAMPLE 1: amelioration of alpha-synuclein neurotoxicity in a Drosophila model

To analyze the capacity of camptothecin, carbenoxolone, and their analogs to prevent neurotoxicity of alpha-synuclein, the Drosophila model is used. Ectopic expression of human SNCA transgenes in Drosophila recapitulate all major pathological features of PD, and is a well established model for synucleinopathy (Haywood and Staveley, 2004; Butler et al., 2012). Transgenic Drosophila expressing wild type SNCA, SNCA A53T, and SNCA A30P are compared to GFP controls in compound administration assays. Amelioration of neuronal degeneration, selective dopaminergic (DA) neuronal degeneration, climbing ability, aSYN aggregation, and progressive retinal tissue degeneration are assessed. Representatives from both carbenoxolone and camptothecin compound classes are tested, in addition to positive controls (see Table 3).

**Table 3: test compounds stocks for reducing aSYN neurotoxicity in neurons.**

| Compound | CAS# | Function | Stock Conc. |
|---|---|---|---|
| 10-hydroxycamptothecin | 19685-09-7 | Topoisomerase 1 inhibitor | 15mM |
| Camptothecin | 7689-03-4 | Topoisomerase 1 inhibitor | 15mM |
| Camptothecin (S,+) | 2112992 | Topoisomerase 1 inhibitor | 2mg/mL |
| Camptothecin 7-Ethyl-10-Hydroxy | 86639-52-3 | Topoisomerase 1 inhibitor | 15mM |
| Topotecan hydrochloride | 119413-54-6 | Topoisomerase 1 inhibitor | 15mM |
| Irinotecan hydrochloride | 100286-90-6 | Topoisomerase 1 inhibitor | 15mM |
| Rubitecan | 91421-42-0 | Topoisomerase 1 inhibitor | 15mM |
| Dexrazoxane | 24584-09-6 | Topoisomerase 2alpha inhibitor | 15mM |
| Etoposide | 33419-42-0 | Topoisomerase 2 inhibitor | 15mM |
| Amsacrine hydrochloride | 54301-15-4 | Topoisomerase 2 inhibitor | 15mM |
| Carbenoxolone disodium | 7241-40-1 | Hydroxysteroid dehydrogenase inhibitor | 15mM |
| 18β-glycyrrhetinic acid | 471-53-4 | Hydroxysteroid dehydrogenase inhibitor | 15mM |
| 18α-glycyrrhetinic acid | 1449-05-4 | Hydroxysteroid dehydrogenase inhibitor | 5mg/mL |
| Glycyrrhizic acid ammonium salt | 53956-04-0 | Hydroxysteroid dehydrogenase inhibitor | 5mg/mL |
| 17-Allylamino-17-demethoxygeldanamycin (17-AAG) | 75747-14-7 | Antibiotic, HSP90 inhibitor | 15mM |
| Radicicol | 12772-57-5 | Antibiotic, HSP90 inhibitor | 5mg/mL |
| Geldanamycin | 30562-34-6 | Antibiotic, HSP90 inhibitor | 5mg/mL |
| Rapamycin | 53123-88-9 | Antibiotic, autophagy inducer | 2.5mg/mL |

### Materials and Methods

**Flies and genetics** All genetic strains are obtained from the Bloomington Stock Center. For retinal degeneration assay, the following genotypes are used: *w1118; UAS-SNCA*/*GMR-GAL4, w1118*; *UAS-SNCA A30P*/*GMR-GAL4; w1118; UAS-SNCA A53T*/*GMR-GAL4,* and *w1118; GMR-GAL4* (control). For the fly head dopaminergic neuron cell biology assay, climbing assay, longevity assay, the following genotypes are used: *ddc-GAL4, UAS-SNCA; ddc-GAL4, UAS-SNCA A53T; ddc-UAS SNCA A30P,* and *ddc-GAL4* (control). For primary cell culture, *elav-GAL4, UAS-SNCA A30P and elav-GAL4* (control) are used. Flies are cultured under standard conditions at 21°C. Experimental compounds diluted in DMSO are added to the standard yeast media at concentrations ranging from 1uM to 100mM. Test compounds include: positive control geldanamycin (Sigma), camptothecin (Sigma), 10-hydroxycamptothecin, irinotecan (Sigma), topotecan (Sigma), etoposide (Sigma), dexrazoxane (Sigma), 18β-glycyrrhetinic acid (Sigma), carbenoxolone (Sigma), 18α-glycyrrhetinic acid (Sigma), glycyrrhizic acid ammonium salt (Sigma), 17-Allylamino-17-demethoxygeldanamycin (17-AAG) (Sigma), geldanamycin (Sigma), and rapamycin (Sigma).

**Neuronal culture** Primary cell cultures are prepared from mid-gastrula stage embryos as described previously (Sepp et al., 2008). Briefly, embryos are collected on agar media plates and dechorionated in 50% bleach for 4min., followed by rinsing with sterile distilled water. Embryos are homogenized in Shields and Sang M3 media (Sigma) in a Dounce homogenizer, and debris are pelleted by centrifugation at 40g for 10 min and then 5 min. Neuroblasts are pelleted by centrifugation at 380G for 10min Cells are resuspended at 1x10⁶ cells/mL in Shields and Sang M3 media supplemented with 10U/mL penicillin, 10 ug/mL streptomycin, and 200ng/mL insulin, and plated on 384-well plates (Corning) at 50 uL/well. Cells are incubated at 21°C. Compound is applied 24 hrs after cells are plated. Stock compounds (15mM) and log dilutions (10⁻¹, 10⁻², 10⁻³, 10⁻⁴, and 10⁻⁵) are prepared in DMSO, then each stock is further diluted 1/10 with media, and 1 uL of this working reagent is applied to culture wells. Cultures are matured for 7 days. Cultures are fixed and stained as described previously (Halter et al., 1995), using pan-neuronal marker anti-elav (1:10, Developmental Studies Hybridoma Bank), and anti-human aSYN (1:1000, Abcam), and dopaminergic neuron marker anti-tyrosine hydroxylase (1:1000, Millipore). Cultures are imaged on a Cellomics robotic fluorescence microscope, and morphologies are quantified with Cellomics ArrayScan algorithms and statistically processed with Prism software.

**Tissue immunolabeling** Immunolabeling of whole-mount adult brains are performed as described previously (Wang et al., 2008). Briefly, age matched adult fly brains from each genotype are dissected in phosphate-buffered saline (PBS) and fixed with 4% paraformaldehyde for 1h, followed by three 15min washes in 0.5% PBT (PBS with 0.5% Triton X-100). Brains are incubated with primary antibody, then secondary antibody, and mounted in Vectashield (Vector Laboratories, Burlingame, CA). Antibodies are: rabbit anti-green fluorescent protein (GFP) (1:1000, Invitrogen), mouse anti-human aSYN (1:1000, LB 509, abcam), and rabbit A11 (1:100, Millipore). Fluorescent Alexa secondaries are used at 1:1000 (Molecular Probes). Brains are imaged on a confocal microscope and analyzed with ImageJ.

**aSYN immunoblots** Fractionations of fly head lysates are carried out as described previously (Chen and Feany, 2005). For each genotype, 350 heads from flies 5 days after eclosion are homogenized at 4°C in buffer A (50mM Tris-HCl, pH 7.5, 1 mM EGTA, 1 mM dithiothreitol with protease inhibitors) and centrifuged at 100,000g for 30 min. Pellets are extracted sequentially by homogenization in Triton-X-100 (buffer A containing 1% TritonX-100, 10% sucrose, and 0.5M sodium chloride), Sarkosyl (50mM Tris-HCl, pH 7.5, 1% Sarkosyl, and 1 mM EGTA), and urea (50mM Tris-HCl, pH 7.5, 8M urea, and 1mM EGTA) followed by centrifugation at 100,000g for 30 min. Sarkosyl-insoluble and urea-soluble pellets (P) and Tris-soluble supernatants (S) are separated by SDS-PAGE and immunoblotted with anti-α-synuclein antibody and anti-β-actin as a loading control. Immunoblots are imaged on a Li-Cor imager.

**Climbing assay** Climbing ability of flies is assessed as previously described (Feany and Bender, 2000; Hegde et al., 2010). Briefly, 20 flies of each genotype are gently tapped to the bottom of a vertical plastic tube (18 cm in length, 2 cm in diameter), and the percentage that are able to climb 15 cm within 30 seconds is determined. Five trials are performed in each experiment at 1-min intervals, and six experiments are carried out for each age-matched genotype.

**Longevity assay** Embryos are collected from transgenic strains and dispensed in 96-well plates containing liquid media culture (200uL/well). Stock compounds (15, 15 x 10⁻², 15 x10⁻⁵mM) prepared in DMSO, are diluted 1/10 with media, and then 1 uL of this working reagent is dispensed in culture wells. Percentage survival of mutant and negative control larvae are recorded on day 4 following compound administration. Data are plotted graphically.

### Retinal morphology analysis

Flies are aged to 1 or 30 days old. Heads are fixed and embedded in epon. Tangential sections (0.5 um thick) are cut and stained with toluidine blue. Ommatidial architecture is examined by light microscopy, digitally photographed, and quantified with ImageJ.

### Statistics

One-way ANOVA with supplementary Newman-Keuls Multiple Comparison test are applied to compare mutant cohorts. Data are presented as mean ± SEM. Significance will be set at P = 0.05. Two tailed student's t-test is used for image feature quantifications and larval survival.

### Results

Compounds that suppress aSYN neurotoxicity in transgenic Drosophila expressing human *SNCA* transgenes will show any or all of: reduced degeneration of the ommatidial array as determined by morphological analysis of retinal sections, restored ability to climb, improved longevity towards wild type, reduction of Lewy body (LB)-like inclusions in dorsomedial brains, improved morphology of dopaminergic neurons in dorsomedial brains, reduction of LB-like inclusions in primary neurons, improved neurite morphology of primary neurons, reduced aggregation of aSYN in primary neurons, reduced aSYN immunofluorescence labeling in primary neurons, and reduced aSYN protein levels in immunoblots.

In primary culture assays, neurons expressing human mutant aSYN A30P show significantly shorter neurite lengths and exhibited excessive branching as compared to wild type controls (compare black and grey bars in graph panels of Figure 1D). Addition of 30 nM topotecan (a camptothecin analog) improved neurite outgrowth compared to DMSO control (Figure D, left graph), while the addition of 30 nM carbenoxolone reduces the excessive branching phenotype (Figure D, right graph). Examples of primary culture phenotypes are in Figure 1A: aSYN expressing neurons with control DMSO vehicle, Figure 1B: aSYN expressing neurons treated with 60 nM carbenoxolone, and Figure 1C: aSYN expressing neurons treated with 60 nM topotecan. In the larval survival assay in liquid media, both the topoisomerase I inhibitor camptothecin-OH at high dose (15 uM), and the topoisomerase II alpha inhibitor dexrazoxane at medium dose (0.15 uM) show improved survival as compared to DMSO vehicle-treated controls (Figure 1E). Compounds that suppress aSYN neurotoxicity in transgenic Drosophila expressing human *SNCA* transgenes will also show any or all of: reduced degeneration of the ommatidial array as determined by morphological analysis of retinal sections, restored ability to climb, improved longevity towards wild type, reduction of Lewy body (LB)-like inclusions in dorsomedial brains, improved morphology of dopaminergic neurons in dorsomedial brains, reduction of LB-like inclusions in primary neurons, improved neurite morphology of primary neurons, reduced aggregation of aSYN in primary neurons, reduced aSYN immunofluorescence labeling in primary neurons, and reduced aSYN protein levels in immunoblots.

### Example 2: administration of a camptothecin analog to ameliorate synucleinopathy phenotypes in rodent models

The administration of topotecan (TPT) will be tested to show its ability to promote proteolytic clearance of mutant aSYN in striatal and nigral neurons of a rat model of Parkinson's disease (PD). Improved clearance of aSYN is detected through immunofluorescence labeling of brain tissue sections as reduced aSYN-positive aggregates, and through immunoblots as reduced aSYN protein levels. Improved clearance of aSYN in animal models correlates with higher neuronal counts and improved neuritic morphology in brain tissue sections, and functional motor improvements in the Cylinder test.

### MATERIALS AND METHODS:

**Animals and Vector Delivery** An AAV1/2-aSYN A53T vector delivery-based over-expression of aSYN in the rat substantia nigra is chosen to model PD, since it replicates progressive nigrostriatal degeneration and basal ganglia-based motor features of PD better than existing stable transgenic models. Furthermore, time of symptom onset is far more predictable, thus reducing experimental noise. Viral vectors will be delivered as using stereotactic injection. Briefly, 20 Sprague Dawley rats (280g, Charles River) will be injected with AAV1/2-aSYN A53T to the experimental hemisphere (right) and AAV1/2-GFP to the control hemisphere (left) using standard stereotaxic procedures and isoflurane/oxygen anesthesia. Vector (2µL) will be delivered by microinjector (Stoelting, Kiel, WI) at 0.2µL/min with coordinates from Bregma: AP, -5.2mm; ML, -2.0mm; DV, -7.5mm. Viral stock concentrations for both AAV1/2 GFP and AAV1/2-aSYN A53T are 1.7x10¹²gp/mL. Animals are housed in pairs at 20°C with a 12hr light/dark cycle with food and water *ad libitum.* Animals will recover and be maintained with regular care for 3 weeks as viral gene expression of aSYN builds to emerging pathological levels as characterized previously.

**Topotecan Treatment On Day 1 of Week 3, rats are fitted with osmotic mini-pumps to administer TPT to the third ventricle. Intracerebral administration is chosen to maximize the amount of drug given to the brain and minimize side effects in peripheral tissues. Using standard stereotaxic procedures, a 22**-gauge guide cannula (Plastics One, Roanoake, VA) is placed into the third ventricle (AP, -1.3mm, ML, +0.0mm; DV -4.6mm with respect to bregma) of isoflurane/oxygen anesthetized rats, and anchored with dental acrylic. Rats are bilaterally infused with TPT or vehicle using a mini-osmotic pulp (Alzet model 2ML2 pump, flow rate 5.0uL/hr, Durect Corp. Cupertino) to the third ventricle, which is delivered for 2 weeks. The pumps are filled with 16.34mM topotecan (TPT) (CPT06, Molcan Corporation) in 50mM tartaric acid with 0.9% saline (delivered at 37.4 µg/h), or vehicle control alone. Upon cessation of drug on Day 7 of Week 4, or at some other time point, rats are sacrificed by an overdose of pentobarbital (1.0mL of 240mg/mL, i.p.) and exsanguinated by transcardial saline perfusion followed by 4% paraformaldehyde.

**Cylinder Test** Immediately before initial viral delivery (Day 1, Week 1), and Days 1 of Weeks 4, 5 and 6, rats are subjected to the Cylinder Test, which is a sensitive behavioral test for motor function on the unilateral AAV1/2-aSYN A53T rat model. The test determines preference for using the left or right forepaw to climb a glass cylinder wall, where normal rats use both paws. Scoring will be conducted over 6 min. and video recorded. Data will be presented as percent asymmetry according to the Wishaw and Kolb equation.**Striatal Neurochemistry** Since the AAV1/2 aSYN A53T model shows a reduction dopamine (DA) levels and TPT is expected to lead to improved DA synthesis, HPLC is used to quantify total DA levels and its metabolites in the striatum. Striatal tissue samples are homogenized and centrifuged, with the supernatant collected for HPLC analysis and the pellet collected for total protein quantification with a Peirce BCA protein assay. DOPAC, dopamine, HVA (homovanillic acid), and catecholamine levels are quantified by HPLC and recorded as ng analyte/mg total protein. For behavior analysis, histology, and striatal neurochemistry, statistical comparisons are conducted first with 1-way ANOVA, with significance at P<0.05. If ANOVA is significant, post-hoc tests are conducted using Tukey's Multiple Comparison test.

**Western blots** Standard Western blot techniques are used on dissected postmortem tissues to determine if aSYN levels decrease in response to TPT treatment. Antibodies used for Westerns include: mouse anti-human aSYN (LB 509, 1:1000, abcam), mouse anti-beta actin (1:1000, LI-COR Biosciences), and anti-mouse IRDye 800CW secondary antibodies (1:10,000, LI-COR Biosciences). Blots are imaged on an Odyssey Quantitative Fluorescence Imaging System. Two-tailed student's t-test is used Western blot quantifications.

**Pharmacokinetic analysis** Postmortem brain samples will be subjected to standard quadrupole mass spectrophotometry analysis to determine brain penetration of TPT into tissue samples.

Histology Striatal and nigral cell morphology will be assessed on cryosections of postmortem tissue. Distribution of aSYN will be assessed with LB509 (anti-aSYN, 1:1000, abcam) and anti-tyrosine hydroxylase (1:500, Millipore). Dopamine neuron counts will be quantified with standard stereology procedures.

**Results** If TPT reduces toxicity associated with aSYN, or increases the clearance of aSYN protein, we would expect to observe any or all of the following: improved forepaw use symmetry in the cylinder test, improved striatal neurochemistry in postmortem tissue analysis, reduced aSYN levels in postmortem brain samples relative to Actin standards, improved neuronal survival in postmortem histology samples as detected by increased neuron counts, neuron morphology assessment, tyrosine hydroxlase and aSYN antibody labeling.

### EXAMPLE 3: administration of carbenoxolone to ameliorate synucleinopathy phenotypes in rodent models.

The administration of carbenoxolone (CBX) to the chronic MPTP/probenocid (MPTPp) mouse model of Parkinson's will be tested to show the neuroprotectivity and amount of decreased dopaminergic cell loss in the substantia nigra as assayed by histology and behavioral readouts.

### MATERIALS AND METHODS:

**Model choice** The chronic MPTPp model (Lau et al., 1990) is selected for the study because it has several neuroendocrine pathologies that occur in PD that are potentially treatable with CBX. Elevated serum cortisol/corticosterone and down-regulated nigrostriatal GR expression (Palhagen et al., 2010)(Ros-Bernal et al., 2011) are seen in both PD and the chronic MPTP model. As an effective inhibitor of the cortisol regulation enzyme HSD1, CBX has the capacity to normalize hypercortisolemia and restore GR expression levels in the substantia nigra. Since chronic cortisol exposure is neurotoxic, and decreased GR expression potentiates dopaminergic cell loss following MPTP exposure (Ros-Bernal et al., 2011) it is expected that CBX administration will protect against MPTP toxicity. Animals will be maintained and treated according to Institutional Animal Care and Use Committee guidelines.

**Mice and Carbenoxolone treatment** To test the neuroprotective capacity of CBX, mice are pre-dosed with CBX for one week, and then exposed to the lesion-inducing MPTP toxin in combination with the adjuvant probenecid. For the study, there are four test groups: wild-type mice, lesion-induced mice, wild-type mice on CBX, and lesion-induced mice on CBX. CBX dosing is administered by i.p. injection at 20 mg/kg, every other day for 2 months. This regime has previously been reported to be non-toxic, and effective at inhibiting the target enzyme HSD1(Jellinck et al., 1993; Takeuchi et al., 2011). The Parkinson's lesion is induced with MPTP/probenecid (20 mg/kg, 250mg/kg respectively) for 5 weeks (one injection every 3.5 days) starting one week after CBX dosing has commenced. Probenecid blocks the rapid clearance of MPTP and its metabolites from the brain, and results in the sustained loss of dopaminergic neurons for at least 6 months following termination of MPTP/probenecid regime. This model is favored over the acute MPTP models because the disease onset is progressive and kills neurons by apoptosis rather than necrosis (Tatton and Kish, 1997), dopaminergic cell loss is more long-lasting, and behavioral tests that are quantifiable and reproducible have been established.

**Phenotypic Characterization** To assay for functional improvement from CBX administration in the chronic MPTP_{P} model, a battery of behavioral tests are conducted including the grid, pole, and reach (vibrissae-evoked forelimb placing) tests. These tests effectively probe dopaminergic pathway integrity, and basal ganglia function and are thus relevant to PD (Meredith and Kang, 2006). In the chronic MPTP_{P} model 95-98% of DA neurons are lost by 3 weeks following the last MPTP/probenocid injection, and there is minimal recovery by 6 months (at 6 months, 76% of DA neurons are still lost); therefore behavioral tests are conducted within this window. Also, delaying behavioral tests to more than 3 days following MPTP treatment ensures that effects do not result from acute pharmacological effects of MPTP/MPP⁺. The experimenter performing the motor activity tests, is blinded to the identity of each treatment group. For the grid test, three parameters of motor function are analyzed (step distance, % wall time, and forepaw faults) as previously described (Tillerson and Miller, 2003). Statistically significant changes in motor performance with these measures are detectable up to 30 days following lesion induction. For the pole test, the total time required to orient and climb down the pole is measured as previously described (Ogawa et al., 1985). The pole test is conducted 4 days following the last MPTP injection as the largest effect size between control and test groups are found within this schedule, and the effect is lost by 60 days post MPTP treatment (Schintu et al., 2009; Luchtman et al., 2012). Lastly, the reach test, which evaluates dopaminergic innervation of the dorsal striatum is conducted. The reach test gives a reliable response weeks after MPTP/probenocid lesion induction (Meredith and Kang, 2006; Schallert, 2006).

**Quantification of substantia nigra dopaminergic pathology** To evaluate whether pretreatment with CBX can protect dopaminergic neurons from MPTP_{P} induced cell death, Tyrosine Hydroxylase immunohistochemistry is conducted on brain cryosections. Mice (n=10/group) are euthanized following the completion of the behavioral studies (1 week after the last MPTP/probenocid injection) and brain tissue is cryosectioned (30 µm). Lesion size is measured by directly counting the number of TH⁺, Nissel⁺ DA neurons in the substantia nigra pars compacta (SNpc). The experimenter is blinded to the identity of the treatment groups at the time of scoring.

Lesion size is also measured indirectly through biochemical methods as previously described (Takeuchi et al., 2011). Striatum extracts are prepared from all test groups (n =5/group) and then dopamine and DOPAC (dopamine metabolite) are quantified with HPLC, and Tyrosine Hydroxylase (TH) is quantified with Western blotting using anti-TH (1:2000, Pelfreez), and anti-β-actin (loading control, 1:4000, Sigma) antibodies. It is expected that dopamine, DOPAC, and TH levels will correlate directly with lesion size.

Alpha-Synuclein (aSYN) levels are evaluated by immunohistochemistry and Western blotting techniques. It is important to evaluate the effect of CBX on aSYN since mutations in its gene *SNCA* cause a familial form of Parkinson's, and it is a significant component of Lewy Bodies. In the chronic MPTP_{P} model there is an increase in aSYN⁺ immunoreactive cells in the SNpc, which does not return to baseline for approximately 2-weeks following lesion induction (Vila et al., 2000). The greatest increase (robust 4-fold) in aSYN⁺ cells occurs 7 days after lesion induction. Therefore, this time point will be selected for conducting Syn⁺, TH⁺ cell counts in the SNpc. This study is conducted in parallel with the TH⁺ cell counts for lesion size measurements described above. As a secondary measure, aSYN mRNA and protein levels are quantified using qPCR and Western blotting techniques. For these studies, midbrain extracts are prepared from all treatment groups (n=5/group) 4 days following MPTP/ lesioning since aSyn upregulation is greatest at this point. aSyn mRNA/protein upregulation is transient, occurring only during 0-4 days post lesioning and then it returns to baseline (Vila et al., 2000; Meredith et al., 2002).

**Toxin metabolism control experiments** Before starting the CBX *in vivo* study, standard control experiments are conducted to ensure that CBX does not interfere with brain MPTP metabolism and therefore lesion induction (Jackson-Lewis and Przedborski, 2007). A single dose of MPTP/probenocid (20 mg/kg, 250mg/kg) is administered to CBX treated (20 mg/kg) and untreated animals. Subsequently MPP⁺ levels are measured by HPLC in the striata (ng/mg tissue) at 90 mins and 3 hours post injection time (n=5 animals/time point and treatment group). These time points take into consideration the fast pharmacokinetics and access to the brain of MPTP, and relatively slower pharmacokinetics of CBX (Jellinck et al., 1993; Dhanesha et al., 2012). If CBX is found to interfere with MPTP metabolism, then the dosing regime is changed such that CBX is only administered after lesion induction. If a neuroprotective effect of CBX is detected at the end of our study, additional control experiments are performed to further rule out any interaction between CBX and MPTP metabolism. For example, experiments are run to test if CBX can inhibit MAO-B, or the dopamine transporter directly as these proteins are important for converting MPTP into the toxic MPP⁺ species, and for uptake into dopaminergic neurons.

**Statistics** Data are subjected to one-way ANOVA (F), when appropriate, with repeated measurements followed by a post hoc Tukey test. Data are presented as mean ± SEM. Significance will be set at P = 0.05.

**Results** If CBX is neuroprotective in the chronic MPTPp mouse model of PD, then we would expect an improvement in basal ganglia function and performance in behavioral assays that probe these circuits (grid, pole and reach tests). In addition we would expect an increase in survival of dopaminergic neurons in the SNpc as demonstrated by more TH⁺ cells, and increased levels dopaminergic cell markers: tyrosine hydroxylase, dopamine and DOPAC. In immunoblots, if CBX is neuroprotective, the levels of DOPAC and TH dopaminergic markers are expected to increase in CBX-MPTPp mice relative to MPTP_{P} controls. Lastly, we would also expect a decrease in the number of aSYN⁺ immunoreactive cells in response to MPTP exposure.

### EXAMPLE 4: amelioration of alpha-synuclein neurotoxicity in Drosophila cell-based and in vivo assays

To analyze the capacity of camptothecin, carbenoxolone, and their analogs to prevent neurotoxicity of alpha-synuclein, the Drosophila model was used. Ectopic expression of human SNCA transgenes in Drosophila recapitulate all major pathological features of PD, and is a well established model for synucleinopathy (Haywood and Staveley, 2004; Butler et al., 2012). Transgenic Drosophila expressing SNCA A30P were compared to GFP controls in compound administration assays in primary neurons and in larvae. Representatives from both carbenoxolone and camptothecin compound classes were tested.

### Materials and Methods

**Flies and genetics** All genetic strains were obtained from the Bloomington Stock Center. For primary cell culture and larval assays, *elav-GAL4, UAS-SNCA A30P and elav-GAL4* (control) stocks were used. Flies are cultured under standard conditions at 21°C. Experimental compounds diluted in DMSO were added to the media at concentrations ranging from 1uM to 100mM. Test compounds included: camptothecin (Sigma), 10-hydroxy-camptothecin (Molcan), topotecan (Molcan), dexrazoxane (Sigma), 18β-glycyrrhetinic acid (Sigma), and carbenoxolone (Sigma).

**Primary neuronal culture** Primary cell cultures were prepared from mid-gastrula stage embryos as described previously (Sepp et al., 2008). Briefly, embryos are collected on agar media plates and dechorionated in 50% bleach for 4min., followed by rinsing with sterile distilled water. Embryos were homogenized in Shields and Sang M3 media (Sigma) in a Dounce homogenizer, and debris pelleted by centrifugation at 40g for 10 min and then 5 min. Neuroblasts were pelleted by centrifugation at 380G for 10min. Cells were resuspended at 1x10⁶ cells/mL in Shields and Sang M3 media supplemented with 10U/mL penicillin, 10 ug/mL streptomycin, and 200ng/mL insulin, and plated on 384-well plates (Corning) at 50 uL/well. Cells were incubated at 21°C. Compound was applied 24 hrs after cells were plated. Stock compounds (15mM) and log dilutions (10⁻¹, 10⁻², 10⁻³, 10⁻⁴, and 10⁻⁵) were prepared in DMSO, then each stock was further diluted 1/10 with media, and 1 uL of this working reagent was applied to culture wells. Cultures were matured for 7 days. Cultures were fixed and stained as described previously (Halter et al., 1995), using pan-neuronal marker anti-GFP (1:1000, Life Technologies), and anti-human aSYN (1:1000, Abcam). Cultures were imaged on a Cellomics robotic fluorescence microscope, and morphologies are quantified with Cellomics ArrayScan algorithms and statistically processed with Prism software.

**Larval Survival assay** Embryos were collected from transgenic strains and dispensed in 96-well plates containing liquid media culture (200uL/well). Stock compounds (15, 15 x 10⁻², 15 x10⁻⁵ mM) prepared in DMSO, were diluted 1/10 with media, and then 1 uL of this working reagent was dispensed in culture wells. Percentage survival of mutant and negative control larvae are recorded on day 4 following compound administration. Data were plotted graphically with Prism software.

### Results

Image examples of primary culture phenotypes are, in Figure 1A: aSYN expressing neurons with control DMSO vehicle, Figure 1B: aSYN expressing neurons treated with 60 nM carbenoxolone, and Figure 1C: aSYN expressing neurons treated with 60 nM topotecan. In primary culture assays, neurons expressing human mutant aSYN A30P showed significantly statistically shorter neurite lengths (Figure 1D) and exhibited excessive branching (Figure IE) as compared to wild type controls (compare black and grey bars in graph panels of Figure 1D,E). Reduced neurite lengths and increased branching is a general feature of poor neuronal health in culture. Addition of 30 nM 10-hydroxy-camptothecin (CPT-OH, a camptothecin analog) improved neurite outgrowth (Figure 1D, compare white and black bars), and the addition of 30 nM carbenoxolone reduced excessive branching compared to DMSO vehicle controls (Figure IE, compare white and black bars). In the larval survival assay in liquid media (Figure 2), both the topoisomerase I inhibitor camptothecin-OH at high dose (15 uM, aSYN CPT-OH hi), and the topoisomerase II alpha inhibitor dexrazoxane at medium dose (0.15 uM, aSYN DEX med) showed improved survival as compared to DMSO vehicle-treated controls (aSYN DMSO).

In postmortem analysis of TPT- and vehicle-treated rat brains, it was found aSYN levels were reduced in the TPT treatment with a dose-dependent trend (Figure 1F). The rat which showed the highest brain concentration of TPT showed approximately a four-fold reduction in aSYN, whereas beta-Actin levels were unchanged (Figure 1F). Reduced neurotoxicity as a result of aSYN clearance may be observed through improved neurite morphology and neuronal survival which are detected through immunolabeling of tissue sections of drug-treated rats as compared to controls. Beneficial effects of aSYN clearance in drug-treated rats may also be observed through improved motor control and diminished paralysis, as compared to controls.

### EXAMPLE 5: administration of a camptothecin analog to ameliorate synucleinopathy phenotypes in rat models

The administration of topotecan (TPT) was tested for its ability to promote proteolytic clearance of mutant aSYN in striatal and nigral neurons of a rat model of Parkinson's disease (PD). Improved clearance of aSYN was detected through immunoblots as reduced aSYN protein levels.

### MATERIALS AND METHODS:

**Animals and Vector Delivery** An AAV1/2-aSYN A53T vector delivery-based over-expression of aSYN in the rat substantia nigra was chosen to model PD synucleinopathy, since it replicates progressive nigrostriatal degeneration and basal ganglia-based motor features of PD better than existing stable transgenic models. Furthermore, time of symptom onset is far more predictable, thus reducing experimental noise. Viral vectors will be delivered as using stereotactic injection. Briefly, 20 Sprague Dawley rats (280g, Charles River) were injected with AAV1/2-aSYN A53T to the experimental hemisphere (right) and AAV1/2-GFP to the control hemisphere (left) using standard stereotaxic procedures and isoflurane/oxygen anesthesia. Vector (2µL) was delivered by microinjector (Stoelting, Kiel, WI) at 0.2µL/min with coordinates from Bregma: AP, -5.2mm; ML, -2.0mm; DV, -7.5mm. Viral stock concentrations for both AAV1/2 GFP and AAV1/2-aSYN A53T were 1.7x10¹²gp/mL. Animals were housed in pairs at 20°C with a 12hr light/dark cycle with food and water *ad libitum.* Animals recovered were maintained with regular care for 3 weeks as viral gene expression of aSYN built to emerging pathological levels as characterized previously.

**Topotecan Treatment** On Day 1 of Week 3, rats were fitted with osmotic mini-pumps to administer TPT to the third ventricle. Intracerebral administration was chosen to maximize the amount of drug given to the brain and minimize side effects in peripheral tissues. Using standard stereotaxic procedures, a 22-gauge guide cannula (Plastics One, Roanoake, VA) was placed into the third ventricle (AP, -1.3mm, ML, +0.0mm; DV -4.6mm with respect to bregma) of isoflurane/oxygen anesthetized rats, and anchored with dental acrylic. Rats were bilaterally infused with TPT or vehicle using a mini-osmotic pulp (Alzet model 2ML2 pump, flow rate 5.0uL/hr, Durect Corp. Cupertino) to the third ventricle, which was delivered for 2 weeks. The pumps were filled with 16.34mM topotecan (TPT) (CPT06, Molcan Corporation) in 50mM tartaric acid with 0.9% saline (delivered at 37.4 µg/h), or vehicle control alone. Upon cessation of drug on Day 7 of Week 4 or at some other time point, rats were sacrificed, by an overdose of pentobarbital (1.0mL of 240mg/mL, i.p.) and exsanguinated by transcardial saline perfusion followed by 4% paraformaldehyde.

**Western blots** Standard Western blot techniques are used on dissected postmortem tissues to determine if aSYN levels decrease in response to TPT treatment. Antibodies used for Westerns include: mouse anti-human aSYN (LB 509, 1:1000, abcam), mouse anti-beta actin (1:1000, LI-COR Biosciences), and anti-mouse IRDye 800CW secondary antibodies (1:10,000, LI-COR Biosciences). Blots are imaged on an Odyssey Quantitative Fluorescence Imaging System. Two-tailed student's t-test is used Western blot quantifications.

**Pharmacokinetic analysis** Postmortem brain samples were subjected to standard quadrupole mass spectrophotometry analysis to determine brain penetration of TPT into tissue samples.

**Results** Quantitative immunoblot and pharmacokinetic analysis of rat brains expressing human mutant aSYN or GFP control showed an inverse relationship between the amount of topotecan (TPT) and aSYN, whereas Actin loading controls were largely unchanged. An immunoblot example of the rat brain aSYN hemisphere with highest TPT levels ('TPT hi', 6809 ng TPT/g tissue), compared to a vehicle treated aSYN hemisphere is shown in Figure 3A. In the 'TPT hi' animal, the level of aSYN was reduced approximately five-fold, whereas beta-Actin levels remained largely unchanged (Figure 3A). Quantification of the immunoblot aSYN protein levels was performed with infrared imaging: three animals with an average TPT brain level of 2725 ng TPT/g tissue are shown (Figure 3B, middle bar) showed an approximate 2-fold reduction in aSYN. The 'TPT hi' animal aSYN quantification levels are also graphed (Figure 3B, right bar).

While the applicant's teachings are described in conjunction with various embodiments, it is not intended that the applicant's teachings be limited to such embodiments. On the contrary, the applicant's teachings encompass various alternatives, modifications, and equivalents, as will be appreciated by those of skill in the art.

The present invention can be described according to the following clauses.
Clause 1. A method of treating a synucleinopathy disease or disorder, comprising:
   administering to a subject having or being at risk of having a synucleinopathy disease or disorder, an effective amount of a compound chosen from the group consisting of camptothecin or a camptothecin analog, 10-hydroxycamptothecin, topotecan, irinotecan, 18β-glycyrrhetinic acid, a 18β-glycyrrhetinic acid analog, carbenoxolone, etoposide a Topoisomerase I inhibitor, a Topoisomerase II inhibitor, a Topoisomerase III inhibitor, a Topoisomerase IIIα inhibitor, and a Topoisomerase IIIβ inhibitor, or a salt, or solvate of any of these compounds, either alone, or in any combination.
Clause 2. The method of clause 1, wherein said compound is camptothecin or a camptothecin analog having the structure of Formula (I): 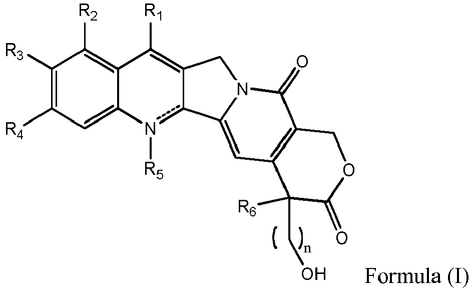 wherein
   R₁ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{A}; =O; -C(=O)R_{A}; -CO₂R_{A}; -CN; -SCN; -SR_{A}; -SOR_{A}; -SO₂R_{A}; -NO₂; -N(R_{A})₂; -NHC(O)R_{A}; or -C(R_{A})₃; wherein each occurrence of R_{A} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   R₂ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{B}; =O; -C(=O)R; -CO₂R_{B}; -CN; -SCN; -SR_{B}; -SORB; -SO₂R_{B}; -NO₂; -N(R_{B})₂; -NHC(O)R_{B}; or -C(R_{B})₃; wherein each occurrence of R_{B} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   R₃ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{C}; =O; -C(=O)R_{C}; -CO₂R_{C}; -CN; -SCN; -SR_{C}; -SOR_{C}; -SO₂R_{C}; -NO₂; -N(R_{C})₂; -NHC(O)R_{C}; or -C(R_{C})₃; wherein each occurrence of R_{C} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   R₄ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{D}; =O; -C(=O)R_{D}; -CO₂R_{D}; -CN; -SCN; -SR_{D}; -SOR_{D}; -SO₂R_{D}; -NO₂; -N(R_{D})₂; -NHC(O)R_{D}; or -C(R_{D})₃; wherein each occurrence of R_{D} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   or wherein R₂ and R₃ together, or R₃ and R₄ together, are -X-(C(R_{E})₂)ₘ-X-, wherein each occurrence of X is independently O, N, or S; each occurrence of R_{E} is independently hydrogen, a protecting group, alkyl, alkoxy, aryloxy, alkylthio, arylthio, amino, alkylamino, dialkylamino, heteroaryloxy, or heteroarylthio; and m is 1, 2, 3, 4, or 5;
   R₅ is hydrogen; cyclic or acyclic, substituted or unsubstituted, branched or unbranched aliphatic; cyclic or acyclic, substituted or unsubstituted, branched or unbranched heteroaliphatic; - C(=O)R_{F}; -CO₂R_{F}; or -C(R_{F})₃; wherein each occurrence of R_{F} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   R₆ is a substituted or unsubstituted, branched or unbranched aliphatic; or cyclic or acyclic, substituted or unsubstituted, branched or unbranched heteroaliphatic;
   n is 0, 1, 2, 3, or 4; or
   a salt, or solvate thereof.
Clause 3. The method of clause 2, wherein R₃ and R₄ are independently hydrogen; hydroxy, halogen; C₁ - C₆ alkyl, -NO₂; -N(R_{C})₂ or -N(R_{D})₂.
Clause 4. The method of either clause 2 or clause 3, wherein n is 0 or 1.
Clause 5. The method of any of clauses 2-4, wherein R₁ is H; a branched or unbranched C₁ - C₆ alkyl which is optionally substituted with one or more amino, halogen, hydroxy, trimethylsilyl, 4 methylpiperazino;

   CH-NO-C(CH₃)₃; or CH₂-CH₂-NH-CH(CH₃)₂.
Clause 6. The method of any of clauses 2-5, wherein R₂ is H; a straight or branched C₁ -C₆ alkyl; or CH₂-N(CH₃)₂.
Clause 7. The method of any of clauses 2-6, wherein R₃ is H; hydroxy; branched or unbranched C₁ - C₆ alkyl; or 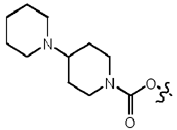
Clause 8. The method of any of clauses 2-7, wherein said compound of Formula (I) has the structure of Formula (II): 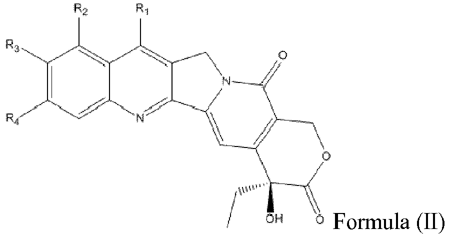 or a salt, or solvate thereof.
Clause 9. The method of clause 1, wherein said compound is a glycyrrhetinic acid or glycyrrhetinic acid analog having the structure: 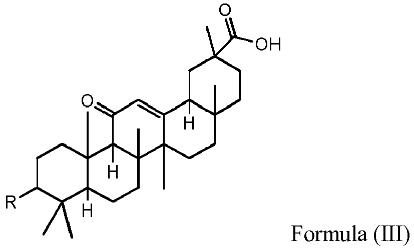 wherein R is OH, -OR_{G}; -CO₂R_{G}; -SO₂R_{G}; wherein each occurrence of R_{G} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio; or a salt or solvate thereof.
Clause 10. The method of clause 9, wherein R is OH, OCO-CH₃, -OCO-(CH₂)₂-COOH, or a salt or solvate thereof.
Clause 11. The method of clause 9 or clause 10, wherein the compound is carbenoxolone or a salt or solvate thereof.
Clause 12. The method of any of the preceding clauses, wherein the synucleinopathy disease or disorder is Parkinson's disease (PD), Parkinson's disease with dementia (PDD), dementia with Lewy bodies (DLB), Lewy body variant of Alzheimer's disease, Alzheimer's disease (AD), Multiple System Atrophy (MSA), Down syndrome, Progressive Supranuclear palsy (PSP), Corticobasal degeneration (CBD), Shy-Drager syndrome, Striatonigral degeneration, Olivopontocerebellar atrophy, Pure autonomic failure, Prion disease, Neurodegeneration with brain iron accumulation type 1 (NBIA1), Frontotemporal dementia (FTD)/Pick's disease, Parkinsonism dementia complex /Amyotrophic lateral sclerosis (PDC/ALS) of Guam, amyotrophic lateral sclerosis (ALS), or a traumatic brain injury.
Clause 13. The method of clause 12, wherein the synucleinopathy disease or disorder is Parkinson's disease.
Clause 14. The method of any of clauses 1-11, wherein the synucleinopathy disease or disorder is a mutation or copy number variation in the human SNCA, LRRK2, PARK2, PARK7, PINK1, Parkin, DJ1, ATP13A2, PLA2G6, FBXO7, UCHL1*,* GIGYF2, HTRA2, EIF4G1, GBA, MAPT, BST1, GAK, APP, PS1, PS2, SOD1, P102L, 6-OPRI, E200K, PLA2G6, PANK2, or FTL gene.
Clause 15. The method of any of clauses 1-11, wherein the synucleinopathy disease or disorder is an aneuploidy of human chromosome 21.
Clause 16. The method of any of clauses 1-11, wherein the subject expresses a mutant protein encoded by the SNCA, LRRK2, PARK2, PARK7, PINK1, Parkin, DJ1, ATP13A2, PLA2G6, FBXO7, UCHL1*,* GIGYF2, HTRA2, EIF4G1, GBA, MAPT, BST1, GAK, APP, PS1, PS2, SOD1, P102L, 6-OPRI, E200K, PLA2G6, PANK2, or FTL gene.
Clause 17. The method of any of clauses 1-16, wherein the subject has been exposed to neurotoxic compounds or elements including paraquat, rotenone, maneb, manganese, 1-methyl 4-phenyl 1,2,3,6-tetrahydropyridine (MPTP), reserpine, thorazine, toluene, n-hexane, carbon disulfide, carbon monoxide, mercury, cyanide, copper, lead, trichloroethylene, perchloroethylene, or 2,4-dichlorophenoxyacetic acid.
Clause 18. The method of any of clauses 1-17, wherein the subject has been exposed to neurotoxic compounds.
Clause 19. The method of any of clauses 1-18, wherein the subject has experienced head trauma.
Clause 20. The method of any of clauses 1-19, wherein said subject is a carrier of a SNCA Rep1 polymorphism.
Clause 21. The method of any of the preceding clauses, wherein said compound is administered orally.
Clause 22. The method of any of the preceding clauses, wherein said compound is administered at a dose of about 1 mg/day to about 1000 mg/day.
Clause 23. The method of any of the preceding clauses, wherein said compound is administered at a dose of about 5mg/day to about 300 mg/day.
Clause 24. The method of any of the preceding clauses, wherein the subject exhibits an elevated glucocorticoid level.
Clause 25. The method of clause 24, wherein the elevated glucocorticoid level is an elevated cortisol level.
Clause 26. The method of clause 25, wherein the elevated cortisol level is a blood plasma concentration of cortisol of more than 350nmol/L.
Clause 27. The method of clause 26, wherein the elevated cortisol level is a blood plasma concentration of cortisol of more than 700nmol/L.
Clause 28. A method of reducing alpha-synucleinopathy aggregation in neural cells comprising exposing said neural cells to a compound chosen from the group consisting of camptothecin, 10-hydroxycamptothecin, topotecan, irinotecan, 18β-glycyrrhetinic acid, glycyrrhetinic acid analog, carbenoxolone, etoposide, a Topoisomerase I inhibitor, a Topoisomerase II inhibitor, a Topoisomerase III inhibitor, a Topoisomerase IIIα inhibitor, or a Topoisomerase IIIβ inhibitor, or a salt, or solvate of any of these compounds, either alone, or in any combination.
Clause 29. The method of clause 28, wherein said exposing step comprises administering the compound to a subject having or being at risk of having a synucleinopathy disease or disorder.
Clause 30. The method of clause 28 or clause 29, wherein said compound is camptothecin or a camptothecin analog having the structure of Formula (I): 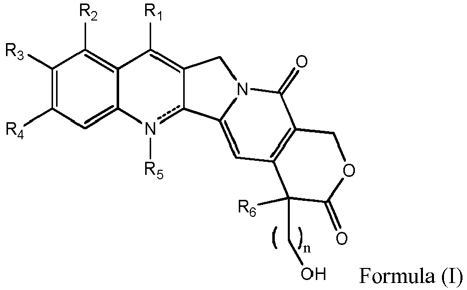 wherein
   R₁ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{A}; =O; -C(=O)R_{A}; -CO₂R_{A}; -CN; -SCN; -SR_{A}; -SOR_{A}; -SO₂R_{A}; -NO₂; -N(R_{A})₂; -NHC(O)R_{A}; or -C(R_{A})₃; wherein each occurrence of R_{A} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   R₂ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{B}; =O; -C(=O)R_{B}; -CO₂R_{B}; -CN; -SCN; -SR_{B}; -SORB; -SO₂R_{B}; -NO₂; -N(R_{B})₂; -NHC(O)R_{B}; or -C(R_{B})₃; wherein each occurrence of R_{B} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   R₃ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{C}; =O; -C(=O)R_{C}; -CO₂R_{C}; -CN; -SCN; -SR_{C}; -SOR_{C}; -SO₂R_{C}; -NO₂; -N(R_{C})₂; -NHC(O)R_{C}; or -C(R_{C})₃; wherein each occurrence of R_{C} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   R₄ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{D}; =O; -C(=O)R_{D}; -CO₂R_{D}; -CN; -SCN; -SR_{D}; -SOR_{D}; -SO₂R_{D}; -NO₂; -N(R_{D})₂; -NHC(O)R_{D}; or -C(R_{D})₃; wherein each occurrence of R_{D} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   or wherein R₂ and R₃ together or R₃ and R₄ together are -X-(C(R_{E})₂)m-X-, wherein each occurrence of X is independently O, N, or S; each occurrence of R_{E} is independently hydrogen, a protecting group, alkyl, alkoxy, aryloxy, alkylthio, arylthio, amino, alkylamino, dialkylamino, heteroaryloxy, or heteroarylthio; and m is 1, 2, 3, 4, or 5;
   R₅ is hydrogen; cyclic or acyclic, substituted or unsubstituted, branched or unbranched aliphatic; cyclic or acyclic, substituted or unsubstituted, branched or unbranched heteroaliphatic; - C(=O)R_{F}; -CO₂R_{F}; or -C(R_{F})₃; wherein each occurrence of R_{F} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   R₆ is a substituted or unsubstituted, branched or unbranched aliphatic; or cyclic or acyclic, substituted or unsubstituted, branched or unbranched heteroaliphatic;
   n is 0, 1, 2, 3, or 4; or
   a salt, or solvate thereof.
Clause 31. The method of any of clauses 28-30, wherein said compound of Formula (I) has the structure of Formula (II): 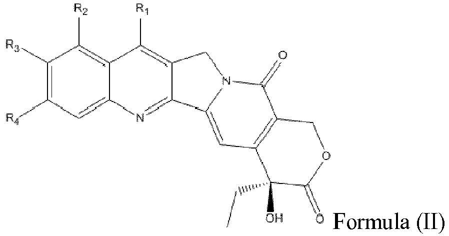 or a salt, or solvate thereof.
Clause 32. The method of clause 31, wherein said compound is a glycyrrhetinic acid or glycyrrhetinic acid analog having the structure: 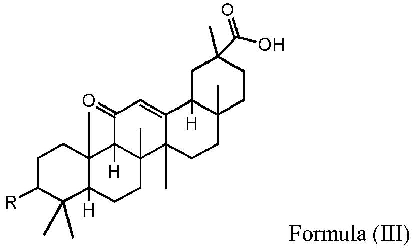 wherein R is OH, -OR_{G}; -CO₂R_{G}; -SO₂R_{G}; wherein each occurrence of R_{G} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   or a salt or solvate thereof.
Clause 33. The method of clause 32, wherein R is OH, OCO-CH₃, -OCO-(CH₂)₂-COOH, or a salt or solvate thereof.
Clause 34. The method of clause 32 or 33, wherein the compound is carbenoxolone or a salt or solvate thereof.
Clause 35. The method of any of clauses 28-34, wherein said alpha-synucleinopathy aggregation in neural cells is reduced by at least 20%.
Clause 36. A method of reducing an elevated glucocorticoid level in neural cells comprising exposing said neural cells to a compound chosen from the group consisting of camptothecin, 10-hydroxycamptothecin, topotecan, irinotecan, 18β-glycyrrhetinic acid, glycyrrhetinic acid analog, carbenoxolone, etoposide, a Topoisomerase I inhibitor, a Topoisomerase II inhibitor, a Topoisomerase III inhibitor, a Topoisomerase IIIα inhibitor, and a Topoisomerase IIIβ inhibitor, or an analog, salt, or solvate of any of these compounds, either alone, or in any combination.
Clause 37. The method of clause 36, wherein said compound reduces said elevated glucocorticoid level to a level observed or expected in healthy neural cells.
Clause 38. The method of clause 36, wherein said elevated glucocorticoid level is a cortisol level.
Clause 39. The method of any of clauses 36-38, wherein said exposing step comprises administering the compound to a subject having elevated glucocorticoid levels.
Clause 40. The method of any of clauses 36-39, wherein said glucocorticoid levels are reduced to a level observed or expected in a healthy subject.
Clause 41. The method of any of clauses 36-40, wherein said elevated glucocorticoid level is a blood plasma concentration of cortisol of more than 350nmol/L.
Clause 42. The method of any of clauses 36-41, wherein said elevated glucocorticoid level is a blood plasma concentration of cortisol of more than 700nmol/L.
Clauses 43. The method of any of clauses 36-42, wherein said compound is camptothecin or a camptothecin analog having the structure of Formula (I): 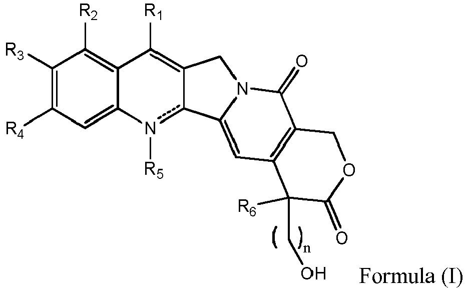 wherein
   R₁ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{A}; =O; -C(=O)R_{A}; -CO₂R_{A}; -CN; -SCN; -SR_{A}; -SOR_{A}; -SO₂R_{A}; -NO₂; -N(R_{A})₂; -NHC(O)R_{A}; or -C(R_{A})₃; wherein each occurrence of R_{A} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   R₂ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{B}; =O; -C(=O)R_{B}; -CO₂R_{B}; -CN; -SCN; -SR_{B}; -SORB; -SO₂R_{B}; -NO₂; -N(R_{B})₂; -NHC(O)R_{B}; or -C(R_{B})₃; wherein each occurrence of R_{B} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   R₃ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{C}; =O; -C(=O)R_{C}; -CO₂R_{C}; -CN; -SCN; -SR_{C}; -SOR_{C}; -SO₂R_{C}; -NO₂; -N(R_{C})₂; -NHC(O)R_{C}; or -C(R_{C})₃; wherein each occurrence of R_{C} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   R₄ is hydrogen; hydroxy; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; -OR_{D}; =O; -C(=O)R_{D}; -CO₂R_{D}; -CN; -SCN; -SR_{D}; -SOR_{D}; -SO₂R_{D}; -NO₂; -N(R_{D})₂; -NHC(O)R_{D}; or -C(R_{D})₃; wherein each occurrence of R_{D} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   or wherein R₂ and R₃ together or R₃ and R₄ together are -X-(C(R_{E})₂)m-X-, wherein each occurrence of X is independently O, N, or S; each occurrence of R_{E} is independently hydrogen, a protecting group, alkyl, alkoxy, aryloxy, alkylthio, arylthio, amino, alkylamino, dialkylamino, heteroaryloxy, or heteroarylthio; and m is 1, 2, 3, 4, or 5;
   R₅ is hydrogen; cyclic or acyclic, substituted or unsubstituted, branched or unbranched aliphatic; cyclic or acyclic, substituted or unsubstituted, branched or unbranched heteroaliphatic; - C(=O)R_{F}; -CO₂R_{F}; or -C(R_{F})₃; wherein each occurrence of R_{F} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   R₆ is a substituted or unsubstituted, branched or unbranched aliphatic; or cyclic or acyclic, substituted or unsubstituted, branched or unbranched heteroaliphatic;
   n is 0, 1, 2, 3, or 4; or
   a salt, or solvate thereof.
Clause 44. The method of clause 43, wherein R₃ and R₄ are independently hydrogen; hydroxy, halogen; C₁ - C₆ alkyl, -NO₂; -N(R_{C})₂ or -N(R_{D})₂.
Clause 45. The method of clause 43 or clause 44, wherein n is 0 or 1.
Clause 46. The method of any of clauses 43-45, wherein R₁ is H; a branched or unbranched C₁ - C₆ alkyl which is optionally substituted with one or more amino, halogen, hydroxy, trimethylsilyl, 4-methylpiperazino; -CH-NO-C(CH₃)₃; or -CH₂-CH₂-NH-CH(CH₃)₂.
Clause 47. The method of any of clauses 43-45, wherein R₂ is H; a straight or branched C₁ - C₆ alkyl; or CH₂-N(CH₃)₂.
Clause 48. The method of any of clasues 43-45, wherein R₃ is H; hydroxy; branched or unbranched C₁ - C₆ alkyl; or 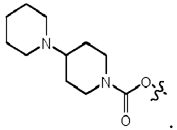
Clause 49. The method of clause 43, wherein said compound of Formula (I) has the structure of Formula (II): 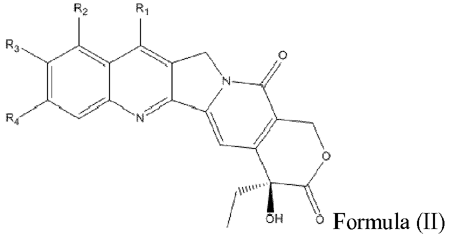 or a salt, or solvate thereof.
Clause 50. The method of clause 43, wherein said compound is a glycyrrhetinic acid or glycyrrhetinic acid analog having the structure: 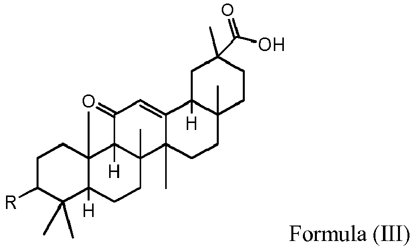 wherein R is OH, -OR_{A}; -CO₂R_{A}; -SO₂R_{A}; wherein each occurrence of R_{A} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;
   or a salt or solvate thereof.
Clause 51. The method of clause 50, wherein R is OH, OCO-CH₃, -OCO-(CH₂)₂-COOH, or a salt or solvate thereof.
Clause 52. The method of clause 50 or 51, wherein the compound is carbenoxolone or a salt or solvate thereof.
Clause 53. The method of any of clauses 36-52, wherein said compound is administered orally.
Clause 54. The method of any of clauses 36-52, wherein said compound is administered at a dose of about 1 mg/day to about 1000 mg/day.
Clause 55. A method of treating a Cushing's Syndrome comprising:
   administering to a subject having or suspected of having Cushing's Syndrome an effective amount of an 18β-glycyrrhetinic acid, an 18β-glycyrrhetinic acid analog, or a salt, or solvate thereof.
Clause 56. A method of treating chronic traumatic encephalopathy comprising:
   administering to a subject having chronic traumatic encephalopathy, an effective amount of a compound chosen from the group consisting of camptothecin or a camptothecin analog, 10-hydroxycamptothecin, topotecan, irinotecan, 18β-glycyrrhetinic acid, a 18β-glycyrrhetinic acid analog, carbenoxolone, etoposide a Topoisomerase I inhibitor, a Topoisomerase II inhibitor, a Topoisomerase III inhibitor, a Topoisomerase IIIα inhibitor, and a Topoisomerase IIIβ inhibitor, or a salt, or solvate of any of these compounds, either alone, or in any combination.

## Claims

1. A compound chosen from the group consisting of glycyrrhetinic acid, a glycyrrhetinic acid analog, carbenoxolone, or a salt, or solvate of any of these compounds, either alone, or in any combination, for use in the treatment or prophylaxis of a synucleinopathy disease or disorder, Cushing's Syndrome, or chronic traumatic encephalopathy; for use in reducing alpha-synucleinopathy aggregation in neural cells; or for use in reducing an elevated glucocorticoid level in neural cells;
wherein said glycyrrhetinic acid or glycyrrhetinic acid analog has the structure: 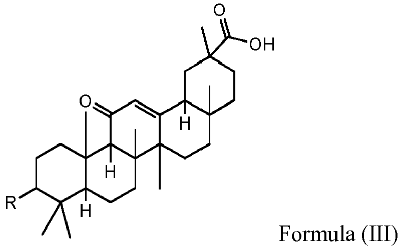 wherein R is OH, -OR_{G}; -CO₂R_{G}; -SO₂R_{G}; wherein each occurrence of R_{G} is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio.

2. The compound, salt or solvate for use of claim 1, wherein R is OH, OCO-CH₃, -OCO-(CH₂)₂-COOH; and/or wherein the compound is carbenoxolone, or a salt or solvate thereof.

3. The compound, salt or solvate for use of claim 1 or claim 2, which is a 18β- glycyrrhetinic acid or a 18β-glycyrrhetinic acid analog or a salt or solvate thereof.

4. The compound, salt or solvate for use of any of the preceding claims, wherein
- the synucleinopathy disease or disorder is Parkinson's disease (PD), Parkinson's disease with dementia (PDD), dementia with Lewy bodies (DLB), Lewy body variant of Alzheimer's disease, Alzheimer's disease (AD), Multiple System Atrophy (MSA), Down syndrome, Progressive Supranuclear palsy (PSP), Corticobasal degeneration (CBD), Shy-Drager syndrome, Striatonigral degeneration, Olivopontocerebellar atrophy, Pure autonomic failure, Prion disease, Neurodegeneration with brain iron accumulation type 1 (NBIA1), Frontotemporal dementia (FTD)/Pick's disease, Parkinsonism dementia complex /Amyotrophic lateral sclerosis (PDC/ALS) of Guam, amyotrophic lateral sclerosis (ALS), or a traumatic brain injury, especially Parkinson's disease;
the synucleinopathy disease or disorder is a mutation or copy number variation in the human *SNCA, LRRK2, PARK2, PARK7, PINK1, Parkin, DJ1, ATP13A2, PLA2G6, FBXO7, UCHL1, GIGYF2, HTRA2, EIF4G1, GBA, MAPT, BST1, GAK, APP, PS1, PS2, SOD1, P102L, 6-OPRI, E200K, PLA2G6, PANK2,* or *FTL* gene;
or
- the synucleinopathy disease or disorder is an aneuploidy of human chromosome 21.

5. The compound, salt or solvate for use of any of claims 1 to 4 for use in the treatment or prophylaxis of a synucleinopathy disease or disorder in a subject whom:
- expresses a mutant protein encoded by the *SNCA, LRRK2, PARK2, PARK7, PINK1, Parkin, DJ1, ATP13A2, PLA2G6, FBXO7, UCHL1, GIGYF2, HTRA2, EIF4G1, GBA, MAPT, BST1, GAK, APP, PS1, PS2, SOD1, P102L, 6-OPRI, E200K, PLA2G6, PANK2,* or *FTL* gene;
- has been exposed to neurotoxic compounds or elements including paraquat, rotenone, maneb, manganese, 1-methyl 4-phenyl 1,2,3,6-tetrahydropyridine (MPTP), reserpine, thorazine, toluene, n-hexane, carbon disulfide, carbon monoxide, mercury, cyanide, copper, lead, trichloroethylene, perchloroethylene, or 2,4-dichlorophenoxyacetic acid;
- has been exposed to neurotoxic compounds;
- has experienced head trauma;
and/or
- is a carrier of a SNCA Rep1 polymorphism.

6. The compound, salt or solvate for use of any of the preceding claims for use in the treatment or prophylaxis of a synucleinopathy disease or disorder in a subject whom exhibits an elevated glucocorticoid level, especially an elevated cortisol level, such as a blood plasma concentration of cortisol of more than 350 nmol/L, or a blood plasma concentration of cortisol of more than 700 nmol/L.

7. The compound, salt or solvate for use of any one of claims 1 to 3 for reducing alpha-synucleinopathy aggregation in neural cells, wherein said alpha-synucleinopathy aggregation in neural cells is reduced by at least 20%.

8. The compound, salt or solvate for use of any one of claims 1 to 3 for reducing an elevated glucocorticoid level in neural cells, wherein said elevated glucocorticoid level reduced to a level observed or expected in healthy neural cells, or wherein said elevated glucocorticoid level is a cortisol level.

9. The compound, salt or solvate for use of any one of claims 1 to 3 or 8 for reducing an elevated glucocorticoid level in neural cells, wherein said glucocorticoid levels are reduced to a level observed or expected in a healthy subject; wherein said elevated glucocorticoid level is a blood plasma concentration of cortisol of more than 350 nmol/L; and/or wherein said elevated glucocorticoid level is a blood plasma concentration of cortisol of more than 700 nmol/L.

10. The compound, salt or solvate for use of any of the preceding claims, wherein:
- said compound, salt or solvate is administered orally;
- said compound, salt or solvate is administered at a dose of about 1 mg/day to about 1000 mg/day;
and/or
- said compound, salt or solvate is administered at a dose of about 5mg/day to about 300 mg/day.
